# EUROPEAN PATENT SPECIFICATION

(11) **EP 3 781 062 B1**
(45) Date of publication and mention of the grant of the patent: **16.08.2023**
(21) Application number: 19718843.6
(22) Date of filing: 16.04.2019
(51) Int. Cl.: A61B 18/18

(54) **MICROWAVE APPLICATOR FOR UTERINE CERVIX**
MIKROWELLENAPPLIKATOR FÜR GEBÄRMUTTERHALS
APPLICATEUR DE MICRO-ONDES POUR COL UTÉRIN

(30) Priority: 16.04.2018 GB 201806193
(43) Date of publication of application: 24.02.2021
(73) Proprietor: Emblation Limited, Inglewood, Alloa FK10 2HU (GB)
(72) Inventor: BEALE, Gary, Alloa FK10 2HU (GB); MCERLEAN, Eamon, Alloa FK10 2HU (GB); KIDD, Matthew Donald, Alloa FK10 2HU (GB); JOSHI, Shailesh, Alloa FK10 2HU (GB)
(74) Representative: Marks & Clerk LLP
(86) International application number: PCT/GB2019/051079
(87) International publication number: WO 2019/202307

(56) References cited:
- WO-A1-99/53853
- CN-B- 104 836 120
- US-A- 4 292 960
- US-A- 5 314 466
- US-A- 6 002 968
- US-A1- 2001 029 368
- US-A1- 2008 314 894
- US-A1- 2010 312 234
- US-A1- 2011 077 634

## Description

### FIELD

The present disclosure relates to a microwave antenna apparatus or applicator, a microwave assembly and a microwave system for use in radiating microwave energy into surface tissue of a uterine cervix in particular, though not exclusively, for use in treating epithelial diseased tissues or conditions affecting tissues such as cervical neoplasia. The present disclosure also relates to methods of fabricating a microwave antenna apparatus or applicator and to methods of radiating microwave energy into surface tissue of a uterine cervix using a microwave antenna apparatus or applicator.

### BACKGROUND

It is known to employ microwave applicators to radiate microwave energy into the surface tissue of a uterine cervix so as to promote heating and activate biological effects in the surface tissue of a uterine cervix, see for instance US 6,002,968.

The exact nature of these biological effects are yet to be quantified, but it may not be heat alone that is responsible for certain biological responses, their magnitude or the time taken for the biological responses to be exhibited. The heat effect is seen when the tissues are in the range of 40°C to 50°C. Without wishing to be bound by theory, it is postulated that non-ablative microwaves energy may up-regulate certain interferon regulated enzymes such as 2'-5'-oligoadenylate synthetase. This is an antiviral enzyme that counteracts viral attack by degrading viral and host RNA. This enzyme utilizes (Adenosine triphosphate) ATP in 2'-specific nucleotidyl transfer reactions to synthesize 2'-5'-oligoadenylates, which upregulate latent ribonuclease (RNASEL), promoting degradation of viral RNA and inhibition of virus replication. The upregulation of RNASEL could be used to therapeutically target selective toxicity against immortalised E6/E7 keratinocytes present in the cervical tissues as one means of non-ablative treatment.

Another limitation to microwave antenna implementation is that any air gap between the dielectric and the target tissue can affect the microwave operating performance unless it has been sufficiently accommodated in the design and controlled in the manufacture by tuning. Air gaps also allow the formation of high order modes and in high power applications can create a source of breakdown causing arcing and burning of the electrode. The effect of air-gaps are particularly relevant if the relative permittivity or epsilon relative (Er) value of the dielectric is much greater than that of the surrounding air (Er 1) such as in high dielectric tissues of Er 20 Er 40 Er 70 etc.

The dielectric properties of the target tissue are a factor in determining the energy needed to achieve the biological response and temperature range (40°C to 50°C). The electrical conductivity of the tissues is another factor. The range of energy needed therefore varies and can be controlled by altering a combination of power (0.1W to 20W) and time (0.1s to 30s).

Cervical intraepithelial neoplasia (CIN) is a common pre-cancerous condition of the cervix associated with human papillomavirus (HPV), which can occur in anyone but is commonly found in younger women who wish to maintain their fertility and treatment often involves surgical excision. The lesion may exist at any one of three stages: CIN1, CIN2, or CIN3 depending on extent of dysplasia: CIN1 being the least (mild) to CIN2 (moderate) and CIN3 the highest (severe). Collectively CIN2+ refers to CIN2 (moderate) and CIN3.

According to the World Health Organization (WHO) guidelines for screening and treatment of precancerous lesions for cervical cancer prevention: supplemental material: GRADE evidence-to-recommendation tables and evidence profiles for each recommendation, 2013, WHO standard practice is to screen women using cytology (Pap test), and when cytology results are positive the diagnosis of CIN is based on subsequent colposcopy, biopsy of suspicious lesions, and then treatment only when CIN2+ has been histologically confirmed. CIN2+ can progress to invasive cancer of the cervix over 10 to 20 years.

The cervix represents the lower cylindrical distal portion of the uterus and is divided into 2 regions: ectocervix and endocervix. The ectocervix is visible during a speculum examination. The endocervix (or endocervical canal) is a luminal cavity within the cervix forming a passageway between the vaginal cavity and the internal os of the cervix. The upper limit of the endocervical canal called the internal os or isthmus and marks the changeover from the endocervix to the endometrium.

Although the initial infection may occur anywhere in the cervical region, the origin of the subsequent dysplasia occurs in the region where cells of two different types: columnar and the squamous epithelium meet. This junction moves during pre-puberty to post-puberty and leaves a "transformation zone".

In transformation zone type 1 (T1) CIN, only the lower outer surface of the ectocervical region is disturbed by the neoplasia; it is fully visible to inspection without manipulation. In transformation zone type 2 (T2) CIN there is a combination of ectocervical and endocervical regions with neoplasia, again visible without manipulation. In transformation zone type 3 (T3) CIN there is a further extension of endocervical component that is beyond the limit of visibility, with manipulation, allowing 3mm of os to be visually exposed.

CIN can be treated with surgery to the cervix either by removal with surgical excision or destruction of the cells covering the cervix such as with ablative therapies such as: laser therapy, heating, or freezing, thermo- and cryo- therapies. While this is effective in the majority of cases, the surgery can cause immediate unwanted effects, such as bleeding and infection, or later complications including difficulty with menses due to scarring of the cervix and early (premature) labour.

Loop electrosurgical excision procedure (LEEP) is a common surgical invasive procedure to treat and manage high-grade cervical premalignant lesions (CIN2+). LEEP is performed with the woman lying back, legs in stirrups, and buttocks at the lower edge of a table (dorsal lithotomy position). A speculum is placed in the vagina and the cervix is the focus for the lighted binocular or monocular microscope (colposcope). In different geographic areas LEEP is also known as Large Loop Excision of the Transformation Zone (LLETZ).

LEEP interventions carry substantial risk of after-effects. Of those treated by LEEP, 67% report pain, 86% bleeding (from 2 to 8 weeks post-intervention) and 65% discharge - see the TOMBOLA (Trial Of Management of Borderline and Other Low-grade Abnormal smears) Group, "After-effects reported by women following colposcopy, cervical biopsies and LLETZ: results from the TOMBOLA trial", BJOG: An International Journal of Obstetrics & Gynaecology, 116: 1506-1514, 2009. Subsequent cauterisation of the LEEP treated area may influence the delayed bleeding incidence and contribute to pain experienced by patients. The most common method of cauterisation is monopolar diathermy where a pad is placed under the patient's buttocks forms a fixed electrode. A second electrode is placed at the desired cautery site where energy is concentrated by the surgical instrument's small surface area. The electrical circuit is completed by passing current through the patient's body. This secondary treatment as part of the LEEP treatment can introduce electrical shocks and burns to the patient inflicting further discomfort - see Cheney, F.W., Posner, K.L., Caplan, R.A. and Gild, W.M., "Burns from warming devices in anesthesia: A closed claims analysis," Anesthesiology, 80(4), pp. 806-810, 1994.

Around 10% of those treated result in a visit to their general practitioner either for reassurance or anti-biotics for suspected infection. Subsequent changes also include 70% experiencing disruption to their menstrual pattern. Lifestyle changes for up to 6 weeks for the patient also include no intercourse or use of tampons.

The effectiveness of LEEP as a CIN treatment has been reported to be 86% to 98% in a 14 month follow up window - see Boonlikit, S. and Srichongchai, H., "Comparison of Recurrence Rates with Contour-Loop Excision of the Transformation Zone (C-LETZ) and Large Loop Excision of the Transformation Zone (LLETZ) for CIN," Asian Pacific Journal of Cancer Prevention, 15(15), pp. 6005-6008, 2014. Over a longer term of 5 years the efficacy may be as low as 43% - see Flannelly, G., Bolger, B., Fawzi, H., Lopes, A. and Monaghan, J.M., "Follow up after LLETZ: could schedules be modified according to risk of recurrence?", BJOG: An International Journal of Obstetrics & Gynaecology, 108(10), pp. 1025-1030, 2001. The eradication of diseased tissue is the objective of the LEEP intervention. The HPV virus is still present in 22% of patients post procedure and forms a contributing factor to poor efficacy rates overall. This rate is similar to the recurrence of warts, another manifestation of HPV infection, when there have been surgical interventions and 30% reappear - see Lipke, M.M., "An armamentarium of wart treatments," Clinical Medicine & Research, 4(4), pp. 273-293, 2006. The HPV particles released during the intervention may also be a contributing factor as presence of HPV in the gaseous plume generated by the LEEP procedure is similar to laser ablative techniques that also require extraction - see Sood, A.K., Bahrani-Mostafavi, Z., Stoerker, J. and Stone, I.K., "Human papillomavirus DNA in LEEP plume," Infectious Diseases in Obstetrics and Gynecology, 2(4), pp. 167-170, 1994.

Cryotherapy, also known as cryosurgery and cryoablation, uses extreme cold to destroy tissues. Ice crystals form in the cells below a certain temperature and cause tearing of the cell membrane, permanently damaging it therefore killing the cell. The source of the low temperature may come from liquid nitrogen or other gasses and may be delivered through a needle to localise the impact. Strict health and safety guidelines are used to ensure no accidental damage occurs to other patient tissues or the operator. Scarring is frequently an additional side effect to this process.

Laser therapy is an alternative ablation technique that may for example use a focused CO₂ laser to burn tissues to their destruction. It is a highly localised technique although the depth of penetration is significantly less than the impact of cryotherapies. The vaporisation action creates an aerosol or plume that must be extracted to prevent secondary infections in the patient and the operator. The power of the laser means the destruction occurs in seconds with the result that accidents can arise from mis-directing the laser onto healthy tissues or the operator's hand. Further operation risks arise because of the ability of the laser to bounce and reflect off reflective surfaces with the result that the procedure can only be carried out in specialised environments.

Is it known to use microwave energy to treat HPV infected tissues. It has been shown that microwave energy can easily penetrate deeply within the epidermal layers to the dermis. The HPV virus is known to reside in the stratum basale and is replicated in the stratum spinosum and stratum granulosum. In an analogous manner, the epithelium layers of the cervix also harbour HPV particles in the basal layer when infected, post abrasion in the upper squamous epithelium.

### SUMMARY

The invention and its preferred embodiments are defined in the appended set of claims.

One of ordinary skill in the art should understand that any of the features of any one of the apparatus, assemblies, systems or methods described herein may apply alone or in any combination in relation to any other one of the apparatus, assemblies, systems or methods described herein.

A microwave antenna apparatus is described herein for use in radiating microwave energy into surface tissue of a uterine cervix, the apparatus comprising:
an electrically conductive ground element defining an aperture;
an electrically conductive elongated element extending through the aperture and terminating at a distal end; and
one or more dielectric elements,
wherein one or more of the dielectric elements electrically insulate the elongated element and the ground element from one another. The one or more of the dielectric elements define an outer surface of the microwave antenna apparatus for engagement with a surface of the uterine cervix, wherein the one or more dielectric elements define a central distal feature for centring the microwave antenna apparatus with respect to an axis of a cervical os of the uterine cervix, and wherein the one or more dielectric elements define a cupping feature for cupping a proximal ectocervix region of the uterine cervix and preventing excessive insertion of the central distal feature into the cervical os of the uterine cervix.

The microwave antenna apparatus may be used to deliver microwave energy to selected areas of the cervix, for example selected areas of the cervix which have been previously identified as exhibiting cervical intraepithelial neoplasia (CIN).

The ground element and the elongated element may be co-axial. This may improve the efficiency of transmission of microwave energy through the microwave antenna apparatus into the surface tissue of the uterine cervix.

One or more of the dielectric elements define an outer surface for engagement with a surface of the uterine cervix.

One or more of the dielectric elements may cover the distal end of the elongated element.

One or more of the dielectric elements may cover a distal portion of the elongated element.

The elongated element may extend axially beyond the ground element by a predetermined length and one or more of the dielectric elements may cover a proportion of the predetermined length of the elongated element.

The elongated element may extend axially beyond the ground element by a predetermined length and one or more of the dielectric elements may cover the whole of the predetermined length of the elongated element.

One or more of the dielectric elements may be configured so as to prevent the elongated element from coming into contact with the surface of the uterine cervix when the microwave antenna apparatus is in use. This may help to avoid burning or charring of the tissue of the surface of the uterine cervix.

One or more of the dielectric elements may be configured to separate the ground element and/or the elongated element from the tissue of the surface of the uterine cervix by a desired predetermined distance. For example, one or more of the dielectric elements may have a desired predetermined thickness.

The one or more dielectric elements may fill the aperture defined by the ground element.

The one or more dielectric elements may define a central distal feature for centring the antenna with respect to an axis of a cervical os of the uterine cervix.

The central distal feature may be configured for radiating microwave energy into a proximal section of the cervical os.

The one or more dielectric elements may define a cupping feature for cupping a proximal ectocervix region of the uterine cervix and preventing excessive insertion of the central distal feature into the cervical os of the uterine cervix.

The microwave antenna apparatus may comprise an electrically conductive cap element at or adjacent the distal end of the elongated conductor. For example, a distal end of an outer surface of the microwave antenna apparatus may be defined by one or more of the dielectric elements and the cap element may be located between the distal end of the elongated conductor and the distal end of the outer surface of the microwave antenna apparatus. Alternatively, the cap element may define the distal end of the outer surface of the microwave antenna apparatus.

One or more of the dielectric elements may cover at least part of the ground element. This may prevent the ground element from coming into contact with the surface of the uterine cervix when the microwave antenna apparatus is in use. This may help to avoid burning or charring of the tissue of the surface of the uterine cervix.

One or more of the dielectric elements may cover at least part of a distal surface of the ground element.

One or more of the dielectric elements may cover at least part of the distal surface of the ground element.

The microwave antenna apparatus may define an axis. For example, the microwave antenna apparatus may be cylindrically symmetrical about an axis.

The elongated element may extend axially beyond the ground element by a predetermined length, for example a predetermined length of less than or equal to 50 mm, between 5 and 15 mm or substantially equal to 10 mm.

The elongated element may be rod-like. The elongated element may be cylindrical. The elongated element may be conical.

A radial extent of the ground element may be more than or less than a radial extent of the dielectric element by a predetermined radial offset. The radial offset may be less than or equal to 20 mm, than or equal to 10 mm or than or equal to 5 mm.

The ground element may be annular or generally annular. The ground element may be planar or generally planar. The ground element may be curved. The ground element may be shaped in the form of a cup or a bowl with an opening of the cup or the bowl directed towards the distal end of the elongated element. The ground element may be shaped in the form of an inverted cup or an inverted bowl with an opening of the inverted cup or the inverted bowl directed away from the distal end of the elongated element.

The elongated element may comprise a proximal portion on an opposite side of the ground element to the distal end of the elongated element.

The ground element may comprise a body portion and an outer conductor portion, wherein the outer conductor portion extends away from the body portion on an opposite side of the body portion to the distal end of the elongated element and wherein the outer conductor portion of the ground element is arranged co-axially with the proximal portion of the elongated element. The microwave antenna apparatus may comprise an electrical connector such as a co-axial electrical connector, wherein the electrical connector is electrically connected to the outer conductor portion of the ground element and to the elongated element.

The microwave antenna apparatus may comprise an outer conductor electrically connected to, and extending away from, the ground element on an opposite side of the ground element to the distal end of the elongated element and wherein the outer conductor is arranged co-axially with the proximal portion of the elongated element. The outer conductor may be soldered or welded to the ground element, the outer conductor may be electrically connected to the ground element using conductive epoxy, and/or the outer conductor and the ground element may be mechanically connected, for example by press-fitting. One or more of the dielectric elements may electrically insulate the outer conductor and the elongated element from one another. The microwave antenna apparatus may comprise an electrical connector such as a co-axial electrical connector, wherein the electrical connector is electrically connected to the outer conductor and the elongated element.

The microwave antenna apparatus may be configured so as to provide a desired predetermined radiation pattern for one or more given characteristics of the microwave energy when the microwave antenna apparatus is located remotely from any other object so that the desired predetermined radiation pattern is unperturbed by the proximity of any other object.

The microwave antenna apparatus may be configured so as to provide a desired predetermined radiation pattern for one or more given characteristics of the microwave energy when the microwave antenna apparatus is located remotely from the surface of the uterine cervix so that the desired predetermined radiation pattern is unperturbed by the proximity of the surface of the uterine cervix.

The microwave antenna apparatus may be configured so as to provide a desired predetermined radiation pattern for one or more given characteristics of the microwave energy when the microwave antenna apparatus is used to apply microwave energy to the uterine cervix.

The microwave antenna apparatus may be configured so as to provide a desired therapeutic effect for one or more given characteristics of the microwave energy when the microwave antenna apparatus is used to apply microwave energy to the uterine cervix.

The microwave antenna apparatus may be configured so as to create the correct biological response for one or more given characteristics of the microwave energy in one or more regions, such as one or more infected regions, of the cervix.

The microwave antenna apparatus may have a relative permittivity which is matched or substantially matched to a relative permittivity of the cervix tissues for one or more given characteristics of the microwave energy. This may improve the efficiency of the transmission of microwave energy to the cervix tissues. This may substantially reduce the risk of heating the microwave antenna apparatus itself and thereby reduce the risk of accidental burning of tissues, should the antenna touch any adjacent tissues immediately post treatment.

The microwave antenna apparatus may have a relative permittivity which differs by less than 50%, less than 10%, less than 1% or less than 0.1% of the relative permittivity of the cervix tissues for one or more given characteristics of the microwave energy.

The microwave antenna apparatus may be configured so as to cause localized non-ablative hyperthermia of the surface tissue of the uterine cervix for one or more given characteristics of the microwave energy.

The microwave antenna apparatus may be configured so as to create a biological response in one or more regions of the surface tissue of a uterine cervix that are infected with human papillomavirus (HPV) and/or diagnosed with cervical intraepithelial neoplasia (CIN) for one or more given characteristics of the microwave energy.

The microwave antenna apparatus may be configured so as to cause localized ablation of the surface tissue of the uterine cervix for one or more given characteristics of the microwave energy.

The microwave antenna apparatus may be configured so as to cauterise the surface tissue of the uterine cervix for one or more given characteristics of the microwave energy.

The one or more given characteristics of the microwave energy may comprise at least one of the frequency, frequency spectrum, power, power density, energy, energy density, intensity, strength, amount, magnitude, exposure time, dose, pulse duration, and pulse repetition rate of the microwave energy.

The one or more given characteristics of the microwave energy may comprise a frequency in the range from about 500 MHZ to about 200 GHz, in the range from about 900 MHz to about 100 GHz or in the range from about 5 GHz to about 15 GHz.

The one or more given characteristics of the microwave energy may comprise a frequency of about 8 GHz.

The one or more given characteristics of the microwave energy may comprise a power of 0.1W to 20W.

The one or more given characteristics of the microwave energy may comprise an exposure time in the range from 0.1s to 30s.

The microwave antenna apparatus may be configured to provide a predetermined radiation pattern for radiating one or more predetermined regions of the surface tissue of the uterine cervix. The microwave antenna apparatus may be configured to provide a radiation pattern for radiating one or more ectocervical regions of the surface tissue of the uterine cervix. The microwave antenna apparatus may be configured to provide a radiation pattern for radiating one or more endocervical regions of the surface tissue of the uterine cervix.

The microwave antenna apparatus may be disposable or re-useable.

The microwave antenna apparatus may comprise a microwave applicator.

There may be provided a plurality of any of the microwave antenna apparatus described above, wherein each microwave antenna apparatus has a different configuration selected to provide a corresponding different radiation pattern. The radiation patterns may, for example, be selected for radiating one or more corresponding ectocervical and/or endocervical regions of the surface tissue of the uterine cervix.

The microwave antenna apparatus may have differently configured outer surfaces. For example, the microwave antenna apparatus may have outer surfaces of different shapes and/or sizes. The microwave antenna apparatus may have differently configured elongated elements and/or ground elements.

One or more of the dielectric materials may include any one or more of acrylonitrile butadiene styrene (ABS), nylon, polyethylene terephthalate (PET), polyimide, polypropylene and polytetrafluoroethylene (PTFE).

A microwave assembly is described herein for use in radiating microwave energy into surface tissue of a uterine cervix, the microwave assembly comprising a shaft connected to any of the microwave antenna apparatus described above.

The microwave assembly may comprise a connection arrangement connecting the shaft and the microwave antenna apparatus, wherein the connection arrangement is configured to vary an angle between the axis of the microwave antenna apparatus and an axis of the shaft.

The connection arrangement may comprise a pivot arrangement, a hinge, a flexible joint, a ball and socket joint, or the like.

Such a connection arrangement may allow the microwave antenna apparatus to be adjusted or oriented for alignment with the cervix entrance or cervical os. For example, such a connection arrangement may allow the angle between the axis of the microwave antenna apparatus and the axis of the shaft to be selected for axial alignment of the microwave antenna apparatus with the cervical os. Changes to the orientation of the microwave antenna apparatus may take place inside the vagina canal by adjusting the angle between the axis of the microwave antenna apparatus and the axis of the shaft after insertion of the microwave antenna apparatus into the vagina. Alternatively, changes to the orientation of the microwave antenna apparatus may take place outside the vagina canal by pre-adjusting the angle between the axis of the microwave antenna apparatus and the axis of the shaft before insertion of the microwave antenna apparatus into the vagina.

The alignment angle may be a fixed angle anywhere between 1 and 90 degrees.

The microwave antenna apparatus may be configured, for example, dimensioned and/or shaped, to be inserted into the vagina and manipulated within the vagina. The microwave antenna apparatus may be configured to be inserted into the vagina and manipulated within the vagina to reach one or more ectocervical and/or endocervical regions of the surface tissue of the uterine cervix. The microwave antenna apparatus may be configured to be inserted into the vagina and manipulated within the vagina by a colposcopist when the patient is in the dorsal lithotomy position.

The connection arrangement may be configured to detachably attach the microwave antenna apparatus to the shaft thereby allowing the fitting of a different microwave antenna apparatus that may feature an alternative configuration, for example an alternative shape and/or size.

The microwave assembly may comprise a hand grip or hand piece at or adjacent a proximal end thereof. For example, the microwave assembly may comprise a hand grip or hand piece at or adjacent a proximal end of the shaft.

The hand grip or hand piece may be detachably attached to the shaft. This may allow the hand grip or hand piece to be detached from the shaft. The microwave antenna apparatus and the shaft may be disposable such and detaching the hand grip or hand piece from the shaft may allow for disposal of the microwave antenna apparatus and the shaft. Alternatively, the microwave antenna apparatus and the shaft may be re-useable and detaching the hand grip or hand piece from the shaft may allow the microwave antenna apparatus and the shaft to be sterilised before re-use.

The hand grip or hand piece and the shaft axis may be co-axial. The hand grip or hand piece may be arranged at an angle relative to the shaft axis. For example, the hand grip or hand piece may be arranged at an angle of 30, 45 or 90 degrees relative to the shaft axis.

The hand grip or hand piece may be disposable or reusable.

The microwave assembly may comprise an electrical switch which is configurable between an on state in which the switch allows the transmission of microwave energy from a microwave generator to the microwave antenna apparatus and an off state in which the switch prevents the transmission of microwave energy from the microwave generator to the microwave antenna apparatus. The hand grip or hand piece may comprise a manual control element such as a button or the like for reconfiguring the electrical switch between the on and off states.

The microwave assembly may comprise an electrical connector such as a coaxial electrical connector to allow the electrical connection of the microwave assembly to a microwave generator through a microwave waveguide. The microwave assembly may comprise an electrical connector such as a coaxial electrical connector to allow the electrical connection of the microwave assembly to a microwave generator through a microwave cable such as a flexible and/or a co-axial microwave cable. The electrical connector may, for example, be electrically connected to the electrical switch.

A microwave system is described herein for use in radiating microwave energy into surface tissue of a uterine cervix, the system comprising:
a microwave generator;
any of the microwave assemblies described above; and
a microwave waveguide,
wherein the microwave waveguide electrically connects the microwave generator and the microwave assembly.

The microwave waveguide may comprise a microwave cable such as a flexible and/or a co-axial microwave cable.

The microwave waveguide may be hardwired, for example soldered or welded or fixed with electrically conductive adhesive, to the microwave assembly.

The microwave waveguide may be housed in the shaft. The shaft may provide mechanical support for the microwave waveguide.

The microwave assembly may comprise an electrical connector such as a coaxial electrical connector, wherein the microwave waveguide is connected electrically to the electrical connector.

The microwave system may comprise a processing resource configured to control the microwave generator to select one or more characteristics of the microwave energy provided by the microwave generator.

The processing resource may be configured to control the microwave generator so as to provide a desired predetermined radiation pattern for one or more given characteristics of the microwave energy when the microwave antenna apparatus is located remotely from any other object so that the desired predetermined radiation pattern is unperturbed by the proximity of any other object.

The processing resource may be configured to control the microwave generator so as to provide a desired predetermined radiation pattern for one or more given characteristics of the microwave energy when the microwave antenna apparatus is located remotely from the surface of the uterine cervix so that the desired predetermined radiation pattern is unperturbed by the proximity of the surface of the uterine cervix.

The processing resource may be configured to control the microwave generator so as to provide a desired predetermined radiation pattern for one or more given characteristics of the microwave energy when the microwave antenna apparatus is used to apply microwave energy to the uterine cervix.

The processing resource may be configured to control the microwave generator so as to provide a desired therapeutic effect for one or more given characteristics of the microwave energy when the microwave antenna apparatus is used to apply microwave energy to the uterine cervix.

The processing resource may be configured to control the microwave generator so as to create the correct biological response for one or more given characteristics of the microwave energy in one or more regions, such as one or more infected regions, of the cervix.

The processing resource may be configured to control the microwave generator so as to cause localized non-ablative hyperthermia of the surface tissue of the uterine cervix for one or more given characteristics of the microwave energy.

The processing resource may be configured to control the microwave generator so as to create a biological response in one or more regions of the surface tissue of a uterine cervix that are infected with HPV and/or diagnosed with CIN for one or more given characteristics of the microwave energy.

The processing resource may be configured to control the microwave generator so as to cause localized ablation of the surface tissue of the uterine cervix for one or more given characteristics of the microwave energy.

The processing resource may be configured to control the microwave generator so as to cauterise the surface tissue of the uterine cervix for one or more given characteristics of the microwave energy.

The one or more given characteristics of the microwave energy may comprise at least one of the frequency, frequency spectrum, power, power density, energy, energy density, intensity, strength, amount, magnitude, exposure time, dose, pulse duration, and pulse repetition rate of the microwave energy.

The one or more given characteristics of the microwave energy may comprise a frequency in the range from about 500 MHZ to about 200 GHz, in the range from about 900 MHz to about 100 GHz or in the range from about 5 GHz to about 15 GHz.

The one or more given characteristics of the microwave energy may comprise a frequency of about 8 GHz.

The one or more given characteristics of the microwave energy may comprise a power of 0.1W to 20W.

The one or more given characteristics of the microwave energy may comprise an exposure time in the range from 0.1s to 30s.

A method is described herein for use in forming any of the microwave antenna apparatus described above.

The method may comprise forming one or more of the dielectric elements of the microwave antenna apparatus by forming one or more of the dielectric materials on, over and/or around any or all of the electrically conductive features of the microwave antenna apparatus. Such a method may result in the electrically conductive features of the microwave antenna apparatus becoming integral to the formed the microwave antenna apparatus thereby avoiding any requirement for assembly of the microwave antenna apparatus.

The method may comprise forming one or more of the dielectric elements using an injection moulding process. The injection moulding process may comprise injection moulding one or more dielectric materials to form the one or more of the dielectric elements. Thus, lower cost and more efficient manufacture may be obtained, particularly when manufacturing large numbers of microwave antenna apparatus and/or shafts such as large numbers of disposable microwave antenna apparatus and/or shafts.

The injection moulding process may comprise forming one or more dielectric materials on, over and/or around the elongated element and/or the ground element.

The injection moulding process may comprise forming one or more dielectric materials on, over and/or around a dielectric material which at least partially covers the elongated element and/or the ground element.

The injection moulding process may comprise forming one or more dielectric materials on, over and/or around an existing component already present in the mould cavity. This process is known as over-moulding. Such an existing component may include any or all of the features of the microwave antenna apparatus.

One or more of the dielectric materials may include any one or more of acrylonitrile butadiene styrene (ABS), nylon, polyethylene terephthalate (PET), polyimide, polypropylene and polytetrafluoroethylene (PTFE).

The method may comprise forming one or more of the dielectric elements using cold compression where a powder form of one or more dielectric materials is compressed into the desired form either by a ram or by an extrusion process. Such a cold compression process may be suitable for PTFE.

The method may comprise forming one or more of the dielectric elements by machining, for example turning a solid blank of dielectric material. Such a method may be appropriate for synthetic fluoropolymer materials such as polytetrafluoroethylene (PTFE) as such materials may not be suitable for traditional injection moulding processes.

The method may comprise affixing the microwave antenna apparatus to the shaft using an adhesive. For example, the method may comprise affixing the microwave antenna apparatus to the shaft using a cyanoacrylate and/or an epoxy adhesive.

The method may comprise affixing the microwave antenna apparatus to the shaft using by thermally fusing the microwave antenna apparatus and the shaft together. Thermal fusing may be an effective bonding method when the microwave antenna apparatus and/or the shaft comprise a synthetic fluoropolymer material.

The method may comprise forming the ground element by at least one of plating, moulding, pressing, 3D printing, attaching to a shaped dielectric, depositing an electrically conductive material onto a 3D surface and the like.

The ground element may comprise or be formed from one or more metals.

The elongated element may comprise or be formed from one or more metals.

The method may comprise forming an electrical connection between an outer conductor of the microwave antenna apparatus and a ground element of the microwave antenna apparatus. The method may comprise soldering or welding the outer conductor and the ground element together. The method may comprise using a conductive epoxy to form the electrical connection between the outer conductor and the ground element. The method may comprise mechanically connecting the outer conductor and the ground element to form the electrical connection between the outer conductor and the ground element for example by press-fitting.

A method is described herein for use in radiating microwave energy into surface tissue of a uterine cervix, the method comprising:
engaging a surface of the uterine cervix with a distal surface of any of the microwave antenna apparatus described above; and
using the microwave antenna apparatus to apply microwave energy to the uterine cervix.

The method may comprise selecting one or more characteristics of the microwave energy so as to provide a desired predetermined radiation pattern when the microwave antenna apparatus is located remotely from any other object so that the desired predetermined radiation pattern is unperturbed by the proximity of any other object.

The method may comprise selecting one or more characteristics of the microwave energy so as to provide a desired predetermined radiation pattern when the microwave antenna apparatus is located remotely from the surface of the uterine cervix so that the desired predetermined radiation pattern is unperturbed by the proximity of the surface of the uterine cervix.

The method may comprise selecting one or more of the frequency, frequency spectrum, power, power density, energy, energy density, intensity, strength, amount, magnitude, exposure time, dose, pulse duration, pulse repetition rate and the like of the microwave energy so as to provide the desired predetermined radiation pattern.

The method may comprise selecting one or more characteristics of the microwave energy so as to provide a therapeutic effect when the microwave antenna apparatus is used to apply microwave energy to the uterine cervix.

The method may comprise selecting one or more characteristics of the microwave energy so as to create the correct biological response in one or more regions, such as one or more infected regions, of the cervix.

The method may comprise matching or substantially matching the relative permittivity of the microwave antenna apparatus to the relative permittivity of the cervix tissues for one or more given characteristics of the microwave energy. This may improve the efficiency of the transmission of microwave energy to the cervix tissues. This may substantially reduce the risk of heating the microwave antenna apparatus itself and thereby reduce the risk of accidental burning of tissues, should the antenna touch any adjacent tissues immediately post treatment.

The method may comprise selecting a relative permittivity of the microwave antenna apparatus which differs by less than 50%, less than 10%, less than 1% or less than 0.1% of the relative permittivity of the cervix tissues for one or more given characteristics of the microwave energy.

The method may comprise selecting one or more of the spatial distribution, frequency, frequency spectrum, power, power density, energy, energy density, intensity, strength, amount, magnitude, exposure time, dose, pulse duration, pulse repetition rate and the like of the microwave energy so as to provide the therapeutic effect.

The method may comprise selecting a frequency of the microwave energy in the range from about 500 MHZ to about 200 GHz, in the range from about 900 MHz to about 100 GHz or in the range from about 5 GHz to about 15 GHz.

The method may comprise selecting a frequency of the microwave energy of about 8 GHz.

The method may comprise selecting a power of the microwave energy of 0.1W to 20W.

The method may comprise selecting an exposure time ranging from 0.1s to 30s.

The method may comprise selecting one or more characteristics of the microwave energy so as to cause localized non-ablative hyperthermia of the surface tissue of the uterine cervix.

The method may comprise selecting one or more of the spatial distribution, frequency, frequency spectrum, power, power density, energy, energy density, intensity, strength, amount, magnitude, exposure time, dose, pulse duration, pulse repetition rate and the like of the microwave energy so as to cause localized non-ablative hyperthermia of the surface tissue of the uterine cervix.

The method may comprise selecting one or more characteristics of the microwave energy so as to create a biological response in one or more regions of the surface tissue of a uterine cervix that are infected with HPV and/or diagnosed with CIN.

The method may comprise selecting one or more of the spatial distribution, frequency, frequency spectrum, power, power density, energy, energy density, intensity, strength, amount, magnitude, exposure time, dose, pulse duration, pulse repetition rate and the like of the microwave energy so as to create a biological response in one or more regions of the surface tissue of a uterine cervix that are infected with HPV and/or diagnosed with CIN.

The method may comprise selecting one or more characteristics of the microwave energy so as to cause localized ablation of the surface tissue of the uterine cervix.

The method may comprise selecting one or more of the spatial distribution, frequency, frequency spectrum, power, power density, energy, energy density, intensity, strength, amount, magnitude, exposure time, dose, pulse duration, pulse repetition rate and the like of the microwave energy so as to cause localized ablation of the surface tissue of the uterine cervix.

The method may comprise selecting one or more characteristics of the microwave energy so as to cauterise the surface tissue of the uterine cervix. Cauterising the surface tissue of the uterine cervix may, for example, be useful following a LEEP.

The method may comprise selecting one or more of the spatial distribution, frequency, frequency spectrum, power, power density, energy, energy density, intensity, strength, amount, magnitude, exposure time, dose, pulse duration, pulse repetition rate and the like of the microwave energy so as to cauterise the surface tissue of the uterine cervix.

### BRIEF DESCRIPTION OF THE DRAWINGS

A microwave antenna apparatus or applicator, a microwave assembly and a microwave system will now be described by way of non-limiting example only with reference to the following figures of which:
FIG. 1 is a schematic illustration of the human cervix;
FIG. 2 is a schematic illustration of a transformation zone type I of the human cervix;
FIG. 3 is a schematic illustration of a transformation zone type II of the human cervix;
FIG. 4 is a schematic illustration of a transformation zone type III of the human cervix;
FIG. 5 is a schematic illustration of a microwave system;
FIG. 6 is an illustration of the external features of a microwave antenna apparatus "type A" designed to be used for the treatment of transformation zone type I (T1) CIN;
FIG. 7 is an illustration of the external features of a microwave antenna apparatus "type B" designed to be used for the treatment of transformation zone type II (T2) CIN;
FIG. 8 is an illustration of the external features of a microwave antenna apparatus "type C" designed to be used for the treatment of transformation zone type III (T3) CIN;
FIG. 9 is an illustration of the external features of a microwave antenna apparatus "type D" which may be a variation of the antenna "type C" designed to be used when dysplasia resides only at the opening of, and continues into, the os of the cervix;
FIG. 10 is an illustration of the external features of a microwave antenna apparatus common to types A, B and C;
FIG. 11 is a cross sectional view of a microwave antenna apparatus type common to types A, B, C and D being applied on the cervix;
FIG. 12 is a cross sectional view of the internal features of a microwave antenna apparatus type common to types A, B, C and D;
FIG. 13 is a representation of the distribution of electromagnetic fields and microwave fields in particular, in a cervix in the form of SAR (surface absorption rate) using a microwave antenna apparatus common to types A, B and C;
FIG. 14 illustrates the scattering parameter S11, a mathematical construct quantifying how RF energy propagates through the microwave antenna apparatus into the tissue;
FIG. 15 is a representation of the distribution of electromagnetic fields and microwave fields in particular, in cervix in the form of SAR (surface absorption rate) using a microwave antenna apparatus common to types A, B and C where the metallic ground plane is in contact with the tissue;
FIG. 16 is a representation of the distribution of electromagnetic fields and microwave fields in particular, in cervix in the form of SAR (surface absorption rate) using a microwave antenna apparatus common to types A, B and C with the metallic ground plane is not in contact with the tissue;
FIG. 17 illustrates the effect of the critical overall length of the central conductor of a microwave antenna apparatus to achieve the overall energy distribution pattern of microwave fields in the tissue confined more towards the ground plane;
FIG. 18 illustrates the effect of the critical overall length of the central conductor of a microwave antenna apparatus to achieve the overall energy distribution pattern of microwave fields in the tissue confined more away from the ground plane;
FIG. 19 illustrates the effect of the critical radial distance from the ground plane to the external form of a microwave antenna apparatus on the overall energy distribution pattern of microwave fields in the tissue;
FIG. 20 illustrates the effect of having equal diameters of the ground plane and the external form of a microwave antenna apparatus on the overall energy distribution pattern of microwave fields in the tissue;
FIG. 21 illustrates the effect of the critical radial distance from the ground plane to the external form of a microwave antenna apparatus on the overall energy distribution pattern of microwave fields in the tissue when the length of the central conductor is extended;
FIG. 22 illustrates the effect of having equal diameters of the ground plane and the external form of a microwave antenna apparatus on the overall energy distribution pattern of microwave fields in the tissue when the length of the central conductor is extended;
FIG. 23 illustrates the effect of a conical ground plane of a microwave antenna apparatus on the overall energy distribution pattern of electromagnetic fields in the tissue;
FIG. 24 illustrates the effect of an inverted cup shaped ground plane of a microwave antenna apparatus on the overall energy distribution pattern of electromagnetic fields in the tissue;
FIG. 25 illustrates the effect of a cup shaped ground plane of a microwave antenna apparatus on the overall energy distribution pattern of electromagnetic fields in the tissue;
FIG. 26 illustrates the overall energy distribution pattern of microwave fields in the tissue with the ground plane of a microwave antenna apparatus not in contact with the tissue;
FIG. 27 illustrates the effect of a longer length of the central conductor of a microwave antenna apparatus showing non-uniform overall energy distribution pattern of microwave fields in the tissue with higher fields towards the endocervix and reduced fields towards the ectocervix;
FIG. 28 illustrates compensating the effect of the extended length of the central conductor of a microwave antenna apparatus by changing the shape of the central conductor to achieve uniform overall energy distribution pattern of microwave fields in the entire cervical region; and
FIG. 29 shows a microwave assembly of the microwave system of FIG. 5.

### DETAILED DESCRIPTION OF THE DRAWINGS

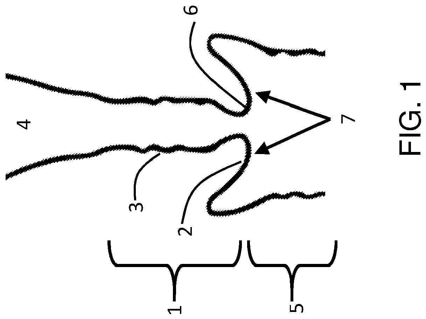

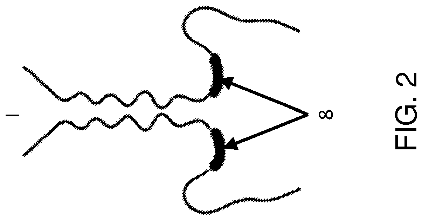

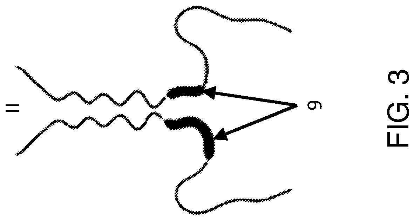

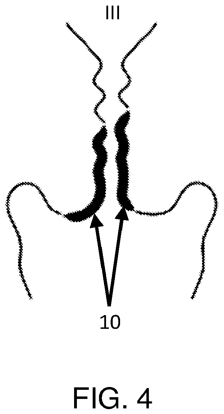

A typical diagrammatic illustration of healthy human cervix is illustrated in the FIG. 1. The cervix 1 comprises an external part in the form of the ectocervix 2 and the endocervix 3, a canal that connects the uterine cavity 4 and the vaginal cavity 5. The opening of the cervix 1 into the vaginal cavity 5 is known as the external os 6. The area where the endocervix 3 meets the ectocervix 2 is called the transformation zone (TZ) 7. This area is the most vulnerable for CIN (cervical neoplasia) and where most abnormalities are thought to arise. FIG. 2 is a schematic illustration of the type I transformation zone (TZ) 8 which is completely ectocervical, is fully visible, and may be small or large. Type II TZ 9 which has an endocervical component but is still fully visible with a small or large ectocervical component is shown in FIG. 3. Type III TZ 10 illustrated in FIG. 4, has an endocervical component, and the upper limit is not fully visible; the ectocervical component, if present, may be small or large.

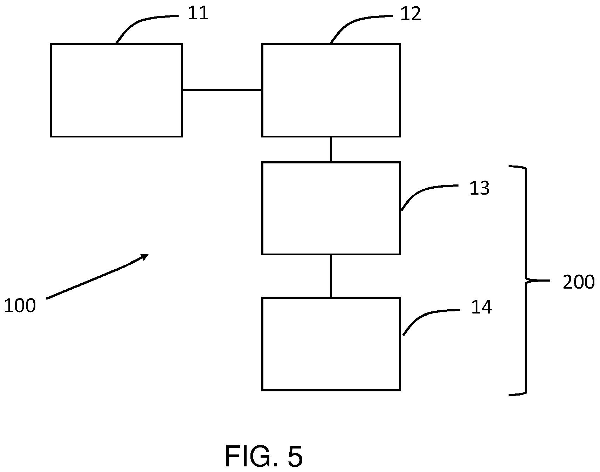

FIG. 5 illustrates a microwave system generally designated 100 for treating the cervix tissue. The microwave system 100 comprises a microwave generator 11 for providing microwave energy, a flexible interconnecting microwave cable such as a co-axial cable 12, a hand grip or hand piece 13, and a microwave antenna apparatus 14.

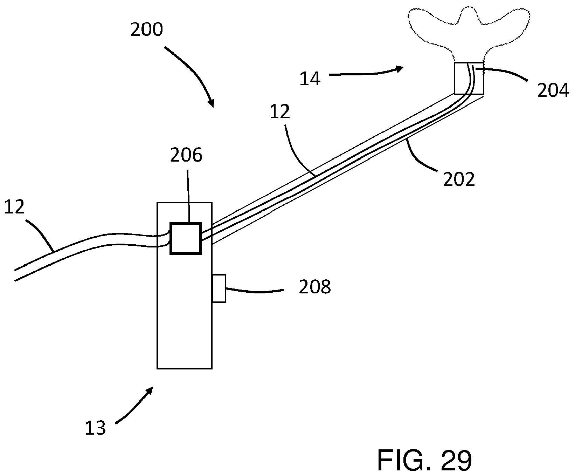

As shown in more detail in FIG. 29, the hand grip or hand piece 13 and the microwave antenna apparatus 14 are connected by a shaft 202. The hand grip or hand piece 13, the microwave antenna apparatus 14 and the shaft 202 together constitute a microwave assembly 200. The microwave assembly 200 is connected to the microwave generator 11 by the co-axial cable 12. The co-axial cable 12 extends through the shaft 202 between the handgrip or hand piece 13 and the microwave antenna apparatus 14. The microwave assembly 200 further comprises a connection arrangement 204 for connecting the microwave antenna apparatus 14 and the shaft 202. The connection arrangement is configured to vary an angle between an axis of the microwave antenna apparatus 14 and an axis of the shaft 202. For example, the connection arrangement 204 may comprise a pivot arrangement, a hinge, a flexible joint, a ball joint or the like. Such a connection arrangement 204 may allow the microwave antenna apparatus 14 to be adjusted or oriented for alignment with the cervix entrance or cervical os. For example, such a connection arrangement 204 may allow the angle between the axis of the microwave antenna apparatus 14 and the axis of the shaft 202 to be selected for axial alignment of the microwave antenna apparatus 14 with the cervical os. Changes to the orientation of the microwave antenna apparatus 14 may take place inside the vagina canal by adjusting the angle between the axis of the microwave antenna apparatus 14 and the axis of the shaft 202 after insertion of the microwave antenna apparatus 14 into the vagina. Alternatively, changes to the orientation of the microwave antenna apparatus 14 may take place outside the vagina canal by pre-adjusting the angle between the axis of the microwave antenna apparatus 14 and the axis of the shaft 202 before insertion of the microwave antenna apparatus 14 into the vagina. For example, the alignment angle may be a fixed angle anywhere between 1 and 90 degrees.

The microwave antenna apparatus 14 may be configured, for example, dimensioned and/or shaped, to be inserted into the vagina and manipulated within the vagina. The microwave antenna apparatus 14 may be configured to be inserted into the vagina and manipulated within the vagina to reach one or more ectocervical and/or endocervical regions of the surface tissue of the uterine cervix. The microwave antenna apparatus 14 may be configured to be inserted into the vagina and manipulated within the vagina by a colposcopist when the patient is in the dorsal lithotomy position.

The connection arrangement 204 may be configured to detachably attach the microwave antenna apparatus 14 to the shaft 202 thereby allowing the fitting of a different microwave antenna apparatus that may feature an alternative configuration, for example an alternative shape and/or size. The connection arrangement 204 may comprise an electrical connector (not shown) such as a coaxial electrical connector to allow the electrical connection/disconnection of the microwave antenna apparatus 14 and the co-axial cable 12.

The hand grip or hand piece 13 may be detachably attached to the shaft 202. This may allow the hand grip or hand piece 13 to be detached from the shaft 202. The hand grip or hand piece 13 may comprise an electrical connector (not shown) such as a coaxial electrical connector to allow the electrical connection/disconnection of the hand grip or hand piece 13 and the co-axial cable 12.

The microwave antenna apparatus 14 and the shaft 202 may be disposable such that detaching the hand grip or hand piece 13 from the shaft 202 may allow for disposal of the microwave antenna apparatus 14 and the shaft 202. Alternatively, the microwave antenna apparatus 14 and the shaft 202 may be re-useable and detaching the hand grip or hand piece 13 from the shaft 202 may allow the microwave antenna apparatus 14 and the shaft 202 to be sterilised before re-use.

The hand grip or hand piece 13 is arranged at an angle relative to an axis of the shaft 202. For example, the hand grip or hand piece 13 may be arranged at an angle of 30, 45 or 90 degrees relative to the axis of the shaft 202, much like a "pistol grip".

The microwave assembly 200 further comprises an electrical switch 206 which is configurable between an on state in which the switch 206 allows the transmission of microwave energy from the microwave generator 11 to the microwave antenna apparatus 14 and an off state in which the switch 206 prevents the transmission of microwave energy from the microwave generator 11 to the microwave antenna apparatus 14. The hand grip or hand piece 13 comprises a manual control element such as a button 208 or the like for reconfiguring the electrical switch 206 between the on and off states.

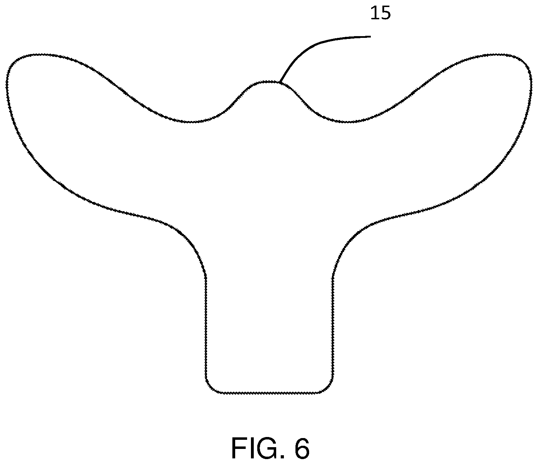

The external form of the microwave antenna apparatus 14 may take a number of different shapes, depending on the desired TZ types and treatment zone for CIN. For the treatment of TZ I shown in FIG. 2, where only the lower outer surface of the ectocervical region is disturbed by the neoplasia 7, the microwave antenna apparatus of type A illustrated in FIG. 6 can be used. The microwave antenna apparatus of type A illustrated in FIG. 6 does not need to treat the cervix os 6. Consequently, the centre feature of the distal end 15 need not be of significant length and need not propagate microwave energy. The purpose of the centre feature 15 is to provide a centring location aid to the user, ensuring a periphery or diameter of the centre feature 15 is in contact with the ectocervix centrally/coaxially.

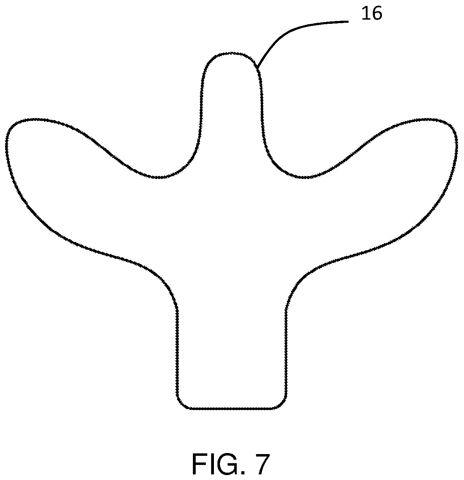

When treating TZ II CIN shown in FIG. 3, there is a combination of visible ectocervical and endocervical regions with neoplasia 8 thus the microwave antenna apparatus of type B illustrated in FIG. 7 features a radiating central feature 16 that serves to not only locate the microwave antenna apparatus relative to the cervix but also to deliver energy to a proximal section of the cervical os 6 for example the first 3mm.

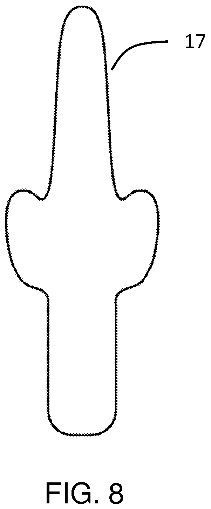

When treating TZ III type CIN shown in FIG. 4, there is a presence of neoplasia 9 in the visible ectocervical region along with visible or non-visible endocervical regions. Consequently, the microwave antenna apparatus of type C FIG. 8 features a radiating central feature 17 that serves to not only locate the microwave antenna apparatus relative to the cervix, but also to deliver energy to the endocervical region that is more distal than that treatable with a type B microwave antenna apparatus.

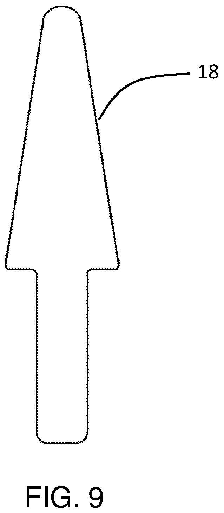

In such situations, because the patient has no or very little ectocervical CIN present and the dysplasia resides only at the opening of, and continues into, the os 6, the microwave antenna apparatus of type D illustrated in FIG. 9 may be used. The microwave antenna apparatus of type D illustrated in FIG. 9 may, for example, be a variation of the microwave antenna apparatus of type C. Here, a type D microwave antenna apparatus comprises a central feature 18 which is configured to radiate microwave energy predominantly in a radial direction.

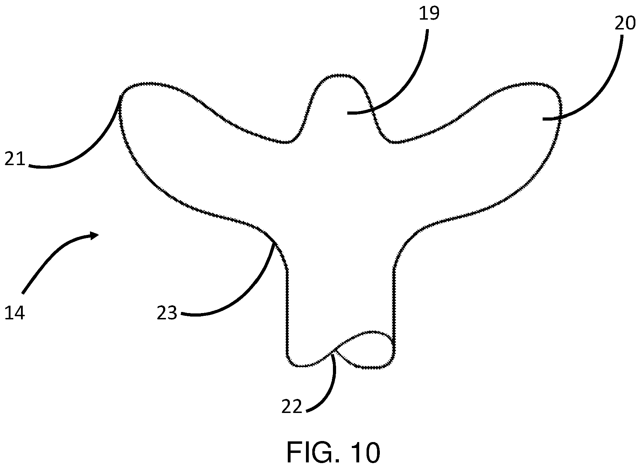

The external features of any type of microwave antenna apparatus 14 are formed to accommodate a range of anatomy types that may vary between patients. Common to types A, B and C is a form illustrated in FIG. 10 that may be tapered or parallel along a major axis, with rounded distal end 19 for centring the antenna with respect to the cervix os 6 axis. Common to types A, B and C is the circular cupping feature 20 that prevents excessive insertion in the os of the cervix 6 and matches the form of a typical proximal ectocervix 2. Also designed to match the typical diameter of this same region is the overall diameter of the antenna 21 which should not interfere with the operation of a speculum and/or a view of the cervix through a colposcope. The approach angle of the microwave antenna apparatus 14 to the cervix is dictated by a combination of an angle of an axis of the shaft 202 of the microwave assembly 200 relative to a patient as controlled by an operator and an angle of an axis of the microwave antenna apparatus 14 as determined by a shaft 22 on which the microwave antenna apparatus 14 is mounted. The approach angle is set such that the microwave antenna apparatus axis and an axis of the os are co-linear. This may serve to ensure that a front-facing distal surface of the microwave antenna apparatus 14 engages the cervix uniformly resulting in uniform exposure to microwave energy and, therefore, uniform treatment. As will be described in more detail below, non-conductive coating 23 covers any exposed metallic components that form the microwave assembly 200. This may reduce the risk of high intensity electromagnetic fields that may lead to an inadvertent burn/deposition of energy when the microwave energy is radiated from the microwave antenna apparatus 14. This coating 23 is made from a biologically compatible substantially electromagnetically transparent material such as polytetrafluoroethylene (PTFE), polyetheretherketone (PEEK) or fluoroethylene polymer (FEP) or other co-polymer coating. The length and diameter of the shaft 202 is designed to allow the user ease of movement when the vagina is opened with a speculum without obscuring the user's view through a colposcope.

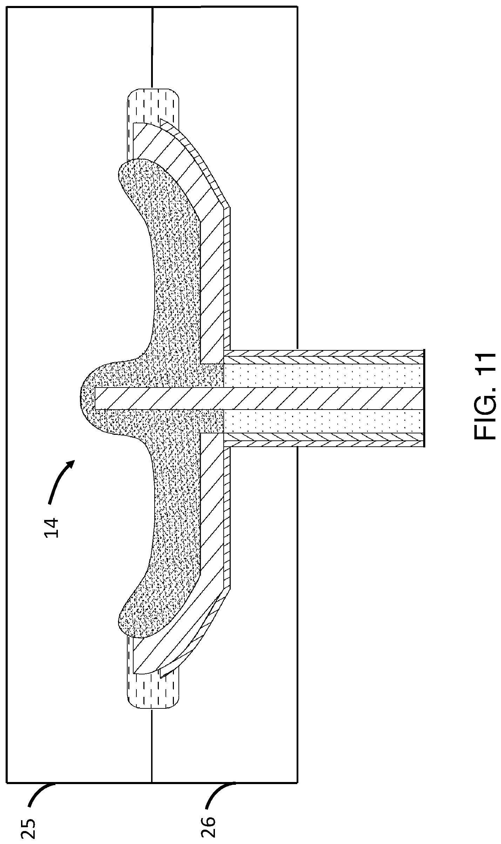

As cross-sectional view of the microwave antenna apparatus 14 common to types A, B and C is shown in the FIG. 11. The antenna is applied on the cervix tissue 25 through air 26.

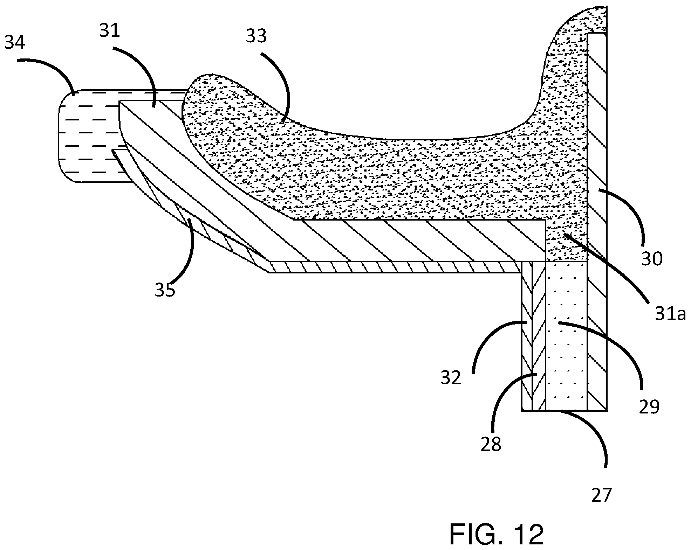

FIG. 12 represents a cross section of a generic microwave antenna apparatus 14 across types A, B and C. The type D microwave antenna apparatus has the same feature as shown in FIG. 12 except for the cupping feature. The microwave antenna apparatus 14 comprises an electrically conductive elongated element in the form of a central conductor 30 and an electrically conductive ground element in the form of an antenna ground plane 31. The antenna ground plane 31 defines an aperture 31a through which the central conductor 30 extends. The microwave antenna apparatus 14 further comprises a dielectric element 33 which covers at least part of a distal or forward-facing surface of the antenna ground plane 31 and which defines an external distal or forward-facing surface or form of the microwave antenna apparatus 14. The dielectric element 33 also insulates the central conductor 30 from the antenna ground plane 31 where the central conductor 30 passes through the aperture of the antenna ground plane 31.

A coaxial cable 27 is formed by an outer conductor in the form of an electrically conductive outer shield 28, a further dielectric element 29, and the central conductor 30. The outer shield 28 is connected to the antenna ground plane 31 with good integrity to ensure all energy is transmitted in to the desired region. Failure to achieve this may result in lower efficiency of energy propagation and non-uniform fields that may result in non-uniform treatment zones. One of ordinary skill in the art will understand that the co-axial cable 12 may extend through the shaft 202 of the microwave assembly 200 and that the central conductor 30 and the outer shield 28 of the microwave antenna apparatus 14 may be electrically connected to a corresponding central conductor (not shown) and a corresponding shield (not shown) of the co-axial cable 12. The microwave antenna apparatus 14 further comprises an insulating support jacket 32 on an exterior surface of the outer shield 28.

A further dielectric element in the form of a dielectric support feature 34 provides additional support and serves to avoid contact between the ground plane 31 and the tissue of the cervix. A yet further dielectric element in the form of an outer jacket 35 provides additional support and insulation for the ground plane 31.

Any one or more of the dielectric elements 32, 33, 34 and 35 may comprise or be formed from any low loss biocompatible material. For example, any one or more of the dielectric elements 32, 33, 34 and 35 may comprise or be formed from at least one of acrylonitrile butadiene styrene (ABS), nylon, polyethylene terephthalate (PET), polyimide, polycarbonate, PC-ABS, polypropylene, ceramics such as alumina and FEP.

Any one or more of the dielectric elements 32, 33, 34 and 35 may comprise or be formed from the same material.

The central conductor 30 and the ground plane 31 may comprise or be formed from a metal such as copper, stainless steel, nickel or the like.

Various different microwave antenna apparatus have been simulated using a 3D simulation model. In this case, the simulation model is HFSS (Ansoft Corp) which is a Finite Element Method (FEM) based full wave electromagnetic solver. Simulations may allow the calculation of a predicted response for coupling efficiency and specific absorption rate (SAR). SAR is a measure of the rate at which energy is absorbed by the human body when exposed to a radio frequency (RF) electromagnetic field.

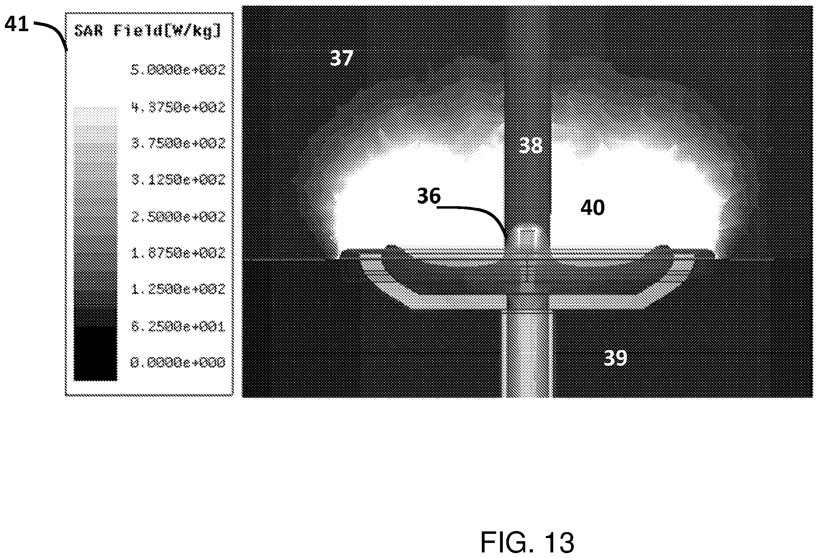

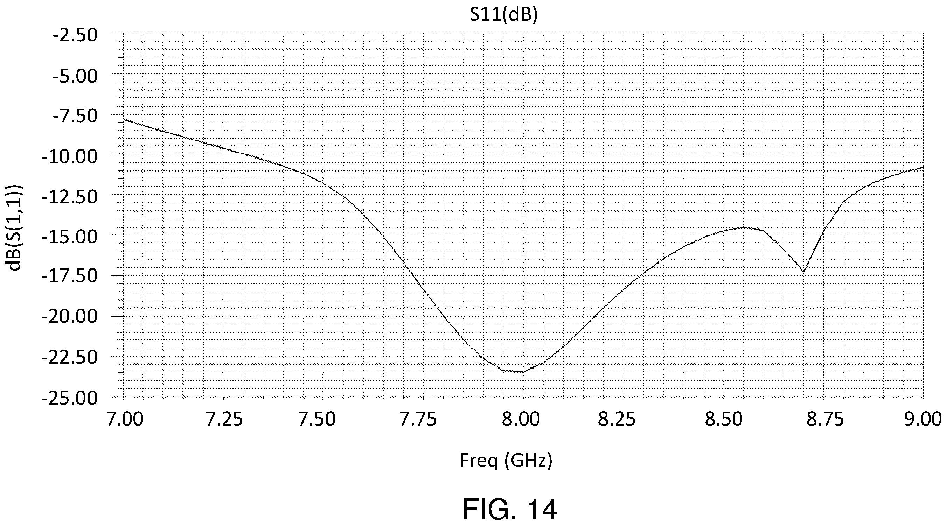

FIG. 13 is a representation of the distribution of the microwave fields using an microwave antenna apparatus 36 into tissue 37 such as cervix tissue. The microwave antenna apparatus 36 fits into the endocervical canal 38 of the cervix. That part of the microwave antenna apparatus 36 which is not in engagement with tissue 37 is modelled into air 39. The SAR field distribution 40 of the microwave fields is illustrated using a greyscale map 41. The scattering parameter S11 response quantifying how RF energy propagates through the microwave antenna apparatus into the tissue is shown FIG. 14. The frequency of operation for microwave fields is plotted on X-axis whereas the S11 or the return loss is shown on the Y-axis in the decibels. The plot shows more than 99% energy being delivered into the tissue at 8 GHz indicating a very good match of the antenna 36 and the tissue 37 at 8 GHz.

The operating frequency of the microwave energy plays a fundamental role in dictating the depth of penetration into the tissue and the overall treatment zone dimensions. 8 GHz is a frequency that may offer a good balance of energy penetration density for a given power. Frequencies less than 8 GHz may penetrate too deeply. Frequencies greater than 8GHz may fail to penetrate deep enough for the correct biological response.

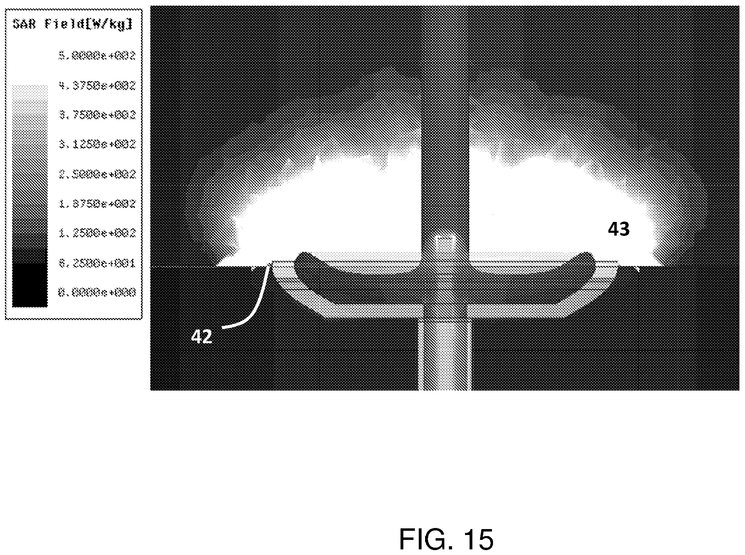

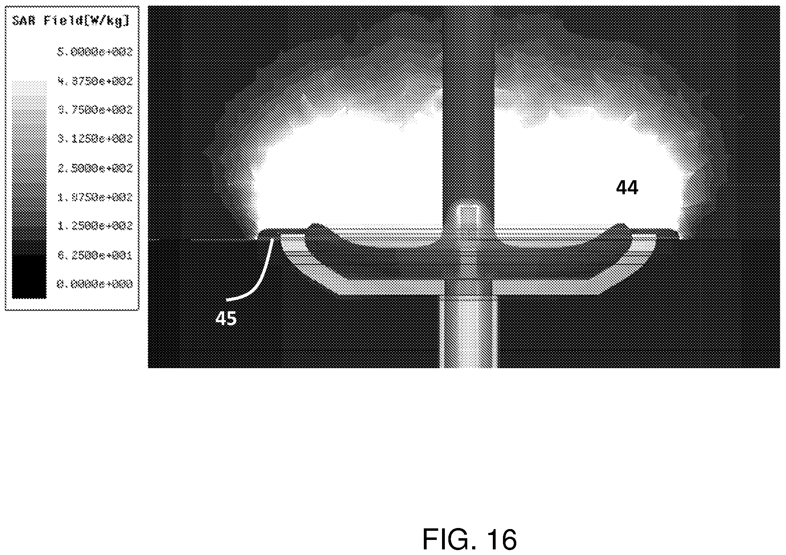

FIG. 15 illustrates the case when the tissue is in contact 42 with the metallic ground plane showing SAR fields 43. The high concentration of the electric fields at the metal-to-tissue interface could cause subsequent charring and burns to the tissue. Consequently, FIG. 16 shows the SAR fields 44 for a microwave antenna apparatus which includes a dielectric element 45 which covers a portion of the metal ground plane so as to avoid any metal-to-tissue interface between the tissue and the metal ground plane and providing a safer solution with SAR fields 44 spread less co-axially as compared with FIG. 15.

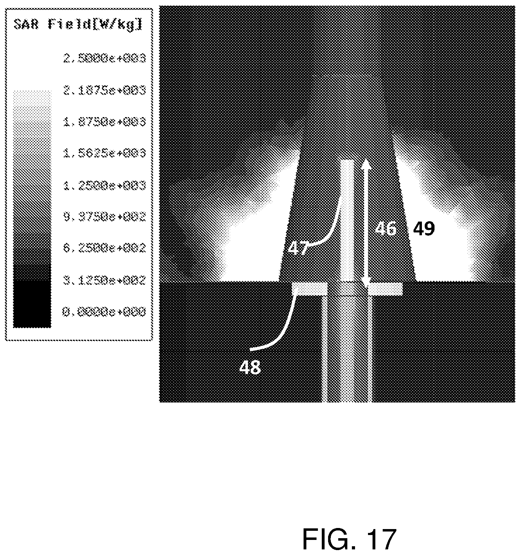

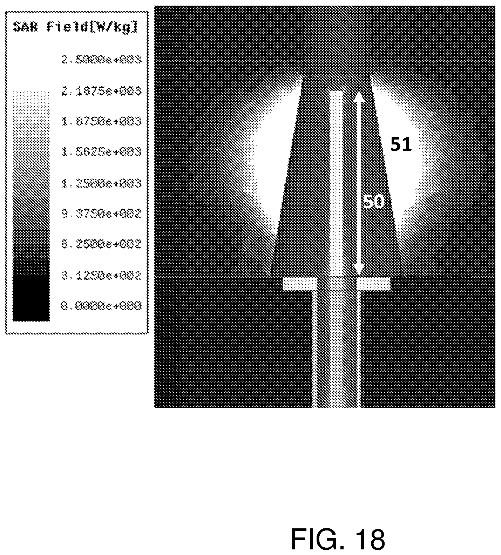

With reference to FIG. 17, the overall length 46 of the central conductor 47 protruding axially from the ground plane 48 is critical to achieving the overall energy transmission pattern into the tissue. In a type D microwave antenna apparatus for example, a central conductor 47 extends from the ground plane 48 for a predetermined length 46. FIG. 17 illustrates the SAR field distribution pattern 49 when the central conductor 47 protrudes axially from the ground plane 48 by 10 mm. The SAR field distribution pattern 49 shows that the fields are stronger towards the ground plane. This could be beneficial to treat the volume into the ectocervical region. In FIG. 18, when the length of the central conductor 50 is 15mm, the fields 51 can be pulled more towards the proximal region or away from the ground plane. This could be used when treating type II or type III TZ CIN having endocervical component.

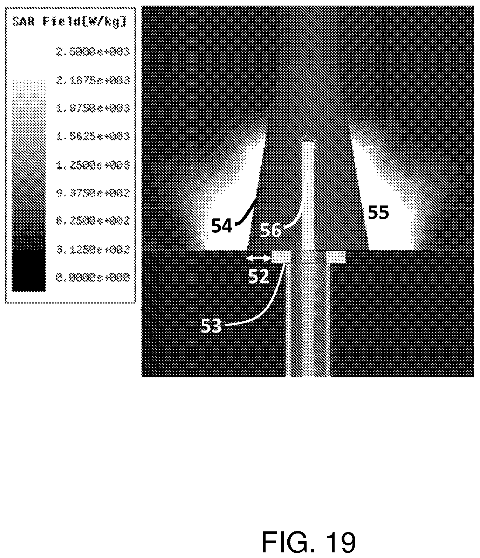

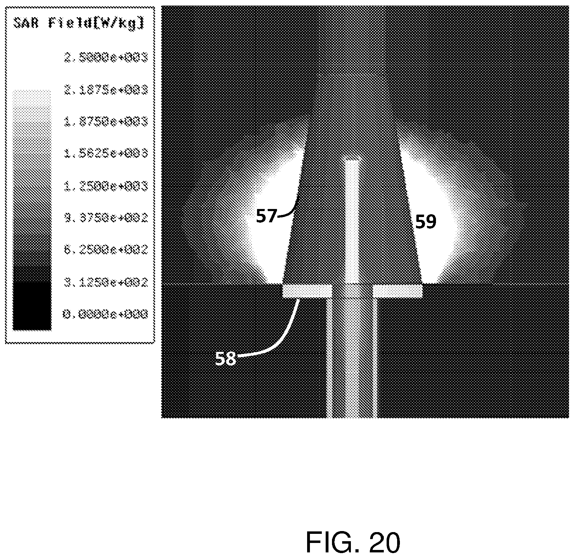

FIG. 19 shows how a radial offset distance 52 between the radial extent of the ground plane 53 and the radial extent or radially outer profile of the external form 54 is critical in achieving the overall stronger distally extended energy distribution pattern 55 into the tissue. The central conductor 56 may be of a certain length such as 10mm. In FIG. 20, the diameter of the external form 57 and the grounding plane 58 are identical which shows the migration of the fields away from the ground plane 59 when compared to the fields 55 shown in FIG. 19. The microwave antenna apparatus of FIG. 20 could be used when treating different types of TZ CIN possessing or not possessing endocervical and ectocervical component.

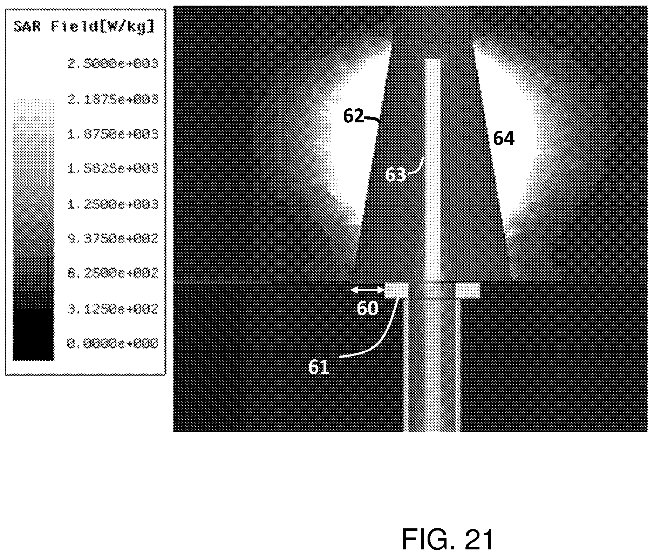

FIG. 21 illustrates how the effects of the varying a radial offset distance 60 between the radial extent of a ground plane 61 and the radial extent of an external form 62 of a microwave antenna apparatus can be compensated by extending the length of the central conductor 56. Specifically, FIG. 21 shows the SAR field for a radial offset 60 between the ground plane 61 and external form 62 which is identical to the radial offset 52, and when the central conductor 63 protrudes further above the ground plane than the central conductor 56 shown in FIG. 19. The fields 64 are confined away from the ground plane 61.

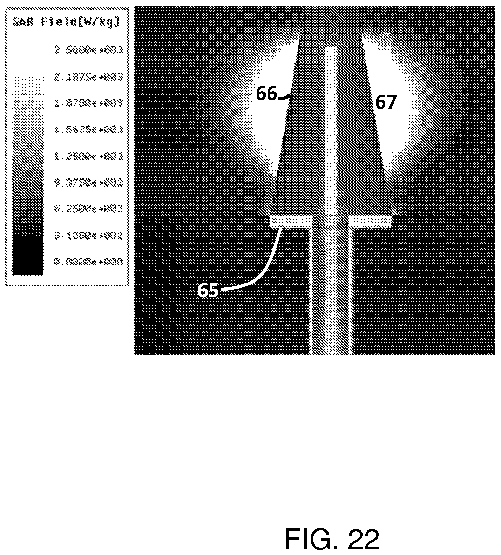

FIG. 22 shows a variation of FIG. 21 without any radial gap between a ground plane 65 and an external form 66. The fields 64 and 67 are almost identical in terms of the distribution region in spite of having different radial distances. This could also mean that when the extended central conductor takes over the field distribution pattern over the radial distance differences between the external form and the ground plane.

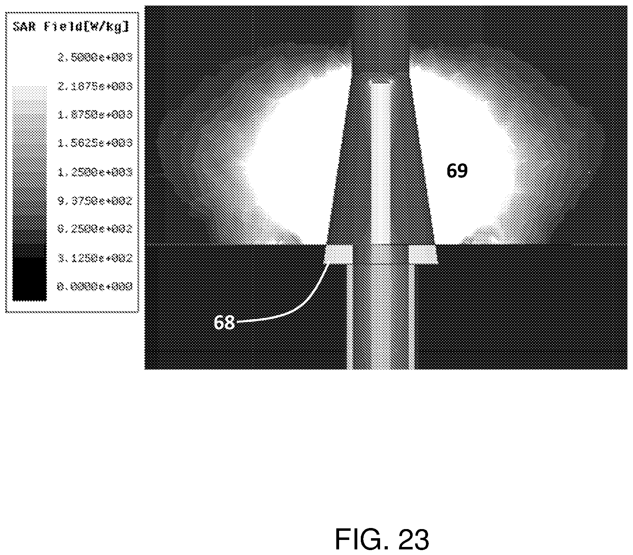

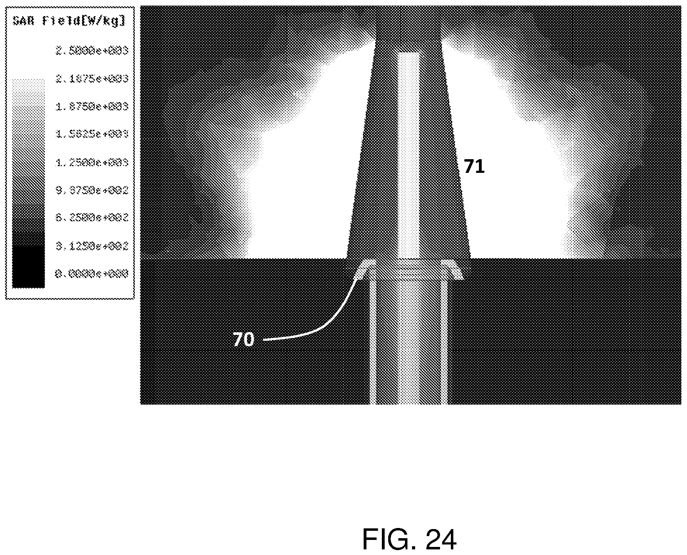

The ground plane can have different shapes to distribute different shapes of the electromagnetic fields into the tissue which in turn could be used to treat different types of TZ neoplasia. For example, a conical shaped ground plane 68 is illustrated in FIG. 23 which may transmit more energy 69 into the endocervical component 3 of the cervix 1, whereas the inverted cup shaped ground plane 70 shown in FIG. 24 could radiate more near the ground plane i.e. radiating the SAR field 71 more into the ectocervical component of the cervix.

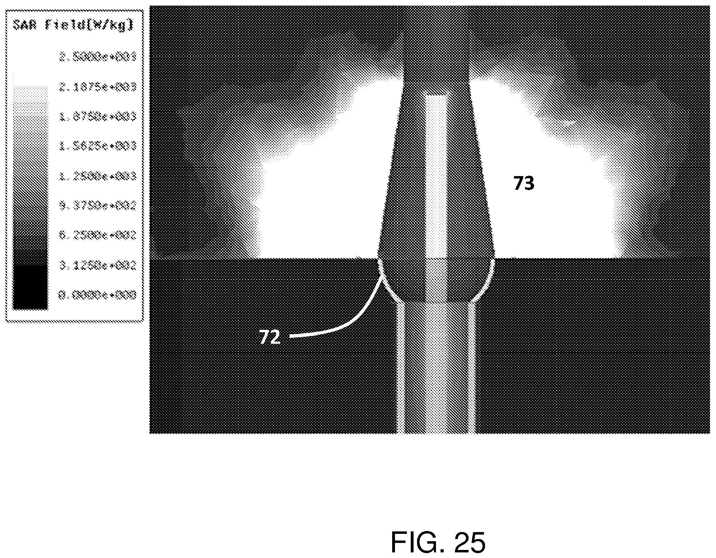

FIG. 25 illustrates another form of ground plane 72 with a cup shaped structure that may spread and pull the radiation 73 further into the ectocervical component of the cervix.

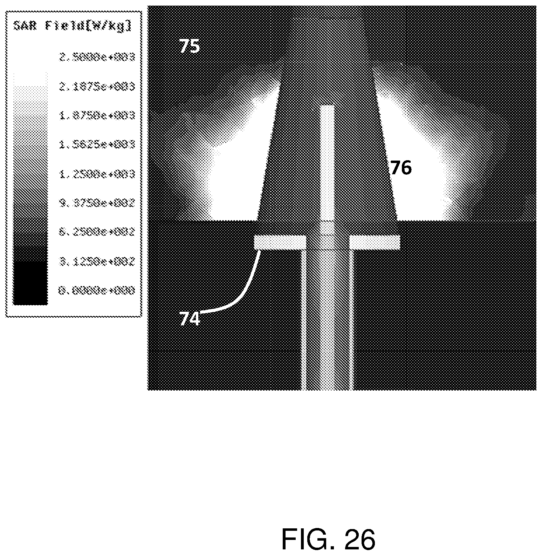

If the ground plane of the microwave antenna apparatus of FIG. 20 makes contact with the tissue, this may result in high electromagnetic fields which could result in charring of the tissue. Alternatively, as illustrated in FIG. 26, the antenna is pulled back away from the tissue avoiding any possibility of the contact of the ground plane 74 with the tissue 75 which shows no change in the radiation field 76 as compared to the 59.

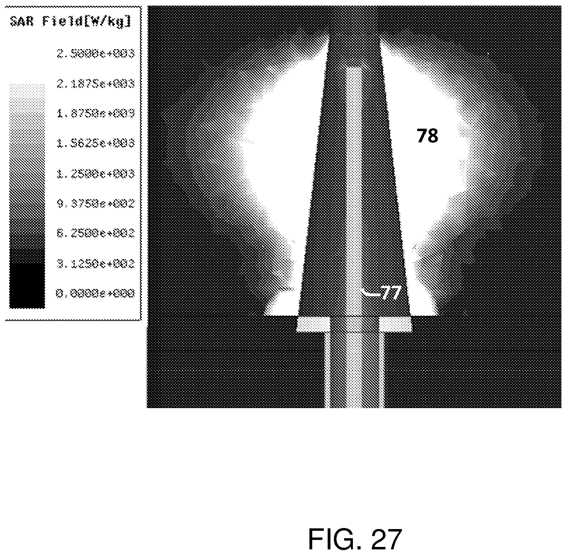

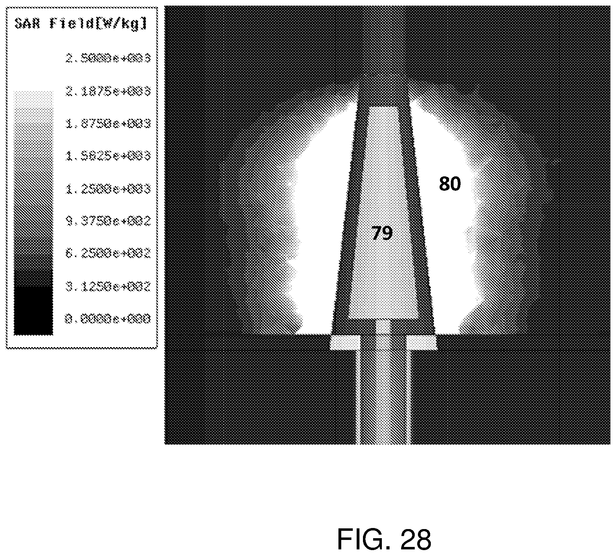

FIG. 27 shows how the shape of the central conductor, in particular of a central conductor of an extended length, may affect the distribution of the fields. FIG. 27 shows a central conductor 77 of length 20 mm showing a non-uniform field pattern 78 which may radiate more into the endocervical component of the cervix. Similarly, with reference to FIG. 28, by thickening the central conductor 79 at the bottom and tapering it towards the top, the fields 80 may be aligned and transmitted uniformly along the entire length of the cervix in advanced cases of CIN.

One of ordinary skill in the art will understand that various modifications may be made to any of the apparatus, assemblies or systems described above. For example, in order to limit the axial propagation of energy, an electrically conductive cap element may be included at or adjacent a distal end of the microwave antenna apparatus. For example, a distal end of an outer surface of the microwave antenna apparatus may be defined by one or more of the dielectric elements and the cap element may be located between the distal end of the elongated conductor and the distal end of the outer surface of the microwave antenna apparatus. Alternatively, the cap element may define the distal end of the outer surface of the microwave antenna apparatus.

The conductive shield of the coaxial cable may comprise two components. For example, the conductive shield of the coaxial cable may comprise a mesh or weave of conductive material and a foil wrap. The foil wrap may supplement the mesh or weave.

The coaxial cable may comprise a coating. The coating may comprise any suitable insulating material, for example PTFR, PEEK, FEB, or parylene. Such a coating may increase robustness. Such a coating may reduce friction to enhance the ease with which the cable may slide relative to another object.

## Claims

1. A microwave antenna apparatus for use in radiating microwave energy into surface tissue of a uterine cervix, the microwave antenna apparatus comprising:
an electrically conductive ground element (31) defining an aperture an electrically conductive elongated element (30) extending through the aperture and terminating at a distal end; and
one or more dielectric elements (29, 32, 33, 34, 35), wherein one or more of the dielectric elements (29, 33) electrically insulate the elongated element (30) and the ground element (31) from one another,
wherein the one or more of the dielectric elements (33, 34) define an outer surface of the microwave antenna apparatus for engagement with a surface of the uterine cervix,
wherein the one or more dielectric elements define a central distal feature for centring the microwave antenna apparatus with respect to an axis of a cervical os of the uterine cervix, and
wherein the one or more dielectric elements (33, 35) define a cupping feature for cupping a proximal ectocervix region of the uterine cervix and preventing excessive insertion of the central distal feature into the cervical os of the uterine cervix.

2. The microwave antenna apparatus as claimed in claim 1, wherein the ground element and the elongated element are co-axial.

3. The microwave antenna apparatus as claimed in any preceding claim, wherein one or more of the dielectric elements cover the distal end of the elongated element and/or wherein one or more of the dielectric elements cover a distal portion of the elongated element.

4. The microwave antenna apparatus as claimed in any preceding claim, wherein the elongated element extends axially beyond the ground element by a predetermined length and, optionally, wherein one or more of the dielectric elements cover a proportion of the predetermined length of the elongated element or one or more of the dielectric elements cover the whole of the predetermined length of the elongated element.

5. The microwave antenna apparatus as claimed in any preceding claim, wherein one or more of the dielectric elements are configured so as to prevent the elongated element from coming into contact with the surface of the uterine cervix when the microwave antenna apparatus is in use.

6. The microwave antenna apparatus as claimed in any preceding claim, wherein one or more of the dielectric elements are configured to separate the ground element and/or the elongated element from the tissue of the surface of the uterine cervix by a desired predetermined distance, for example wherein one or more of the dielectric elements have a desired predetermined thickness.

7. The microwave antenna apparatus as claimed in any preceding claim, wherein the one or more dielectric elements fill the aperture defined by the ground element.

8. The microwave antenna apparatus as claimed in claim 1, wherein the central distal feature is configured for radiating microwave energy into a proximal section of the cervical os.

9. The microwave antenna apparatus as claimed in any preceding claim, wherein one or more of the dielectric elements cover at least part of the ground element and/or wherein one or more of the dielectric elements cover at least part of a distal surface of the ground element.

10. The microwave antenna apparatus as claimed in any preceding claim, wherein the microwave antenna apparatus defines an axis and the microwave antenna apparatus is cylindrically symmetrical about the axis and, optionally, wherein the elongated element is rod-like, cylindrical and/or conical and, optionally, wherein a radial extent of the ground element is more than or less than a radial extent of the one or more dielectric elements by a predetermined radial offset and, optionally, wherein at least one of:
the ground element is annular or generally annular;
the ground element is planar or generally planar;
the ground element is curved;
the ground element is shaped in the form of a cup or a bowl with an opening of the cup or the bowl directed towards the distal end of the elongated element; and
the ground element is shaped in the form of an inverted cup or an inverted bowl with an opening of the inverted cup or the inverted bowl directed away from the distal end of the elongated element.

11. The microwave antenna apparatus as claimed in any preceding claim, comprising an electrically conductive cap element at or adjacent the distal end of the elongated conductor and, optionally, wherein a distal end of an outer surface of the microwave antenna apparatus is defined by one or more of the dielectric elements and the electrically conductive cap element is located between the distal end of the elongated conductor and the distal end of the outer surface of the microwave antenna apparatus.

12. The microwave antenna apparatus as claimed in any preceding claim, wherein the microwave antenna apparatus is configured for use in radiating microwave energy into the surface tissue of the uterine cervix for at least one of:
so as to provide a therapeutic effect in one or more regions, such as one or more regions of the cervix infected with human papillomavirus (HPV) and/or diagnosed with cervical intraepithelial neoplasia (CIN);
so as to create the correct biological response in one or more regions, such as one or more regions of the cervix infected with human papillomavirus (HPV) and/or diagnosed with cervical intraepithelial neoplasia (CIN);
localized non-ablative hyperthermia of the surface tissue of the uterine cervix;
localized ablation of the surface tissue of the uterine cervix; and
cauterisation of the surface tissue of the uterine cervix.

13. A plurality of microwave antenna apparatus as claimed in any preceding claim, wherein each microwave antenna apparatus has a different configuration selected to provide a corresponding different radiation pattern.

14. A microwave assembly for radiating microwave energy into surface tissue of a uterine cervix, the microwave assembly comprising the microwave antenna apparatus as claimed in any preceding claim and a shaft connected to the microwave antenna apparatus and, optionally, comprising a connection arrangement connecting the shaft and the microwave antenna apparatus, wherein the connection arrangement is configured to vary an angle between an axis of the microwave antenna apparatus and an axis of the shaft and, optionally, wherein the connection arrangement comprises at least one of a pivot arrangement, a hinge, a flexible joint, and a ball and socket joint and, optionally, wherein the connection arrangement is configured to detachably attach the microwave antenna apparatus to the shaft thereby allowing the fitting of a different microwave antenna apparatus to the shaft, wherein the different microwave antenna apparatus features an alternative configuration, for example an alternative shape and/or size, to the microwave antenna apparatus and, optionally, wherein the shaft is disposable or the shaft is re-useable and, optionally, comprising a hand grip or hand piece at or adjacent a proximal end thereof, for example, at or adjacent a proximal end of the shaft and, optionally, wherein the hand grip or hand piece is detachably attached to the shaft and, optionally, wherein the hand grip or hand piece is disposable or reusable and, optionally, comprising an electrical switch which is configurable between an on state in which the switch allows the transmission of microwave energy from a microwave generator to the microwave antenna apparatus and an off state in which the switch prevents the transmission of microwave energy from the microwave generator to the microwave antenna apparatus and, optionally, wherein the hand grip or hand piece comprises a manual control element such as a button or the like for reconfiguring the electrical switch between the on and off states.

15. A microwave system for radiating microwave energy into surface tissue of a uterine cervix, the system comprising:
a microwave assembly as claimed in any one of claims 1 to 14;
a microwave generator; and
a microwave waveguide,
wherein the microwave waveguide electrically connects the microwave generator and the microwave assembly and, optionally, wherein the microwave waveguide comprises a microwave cable such as a flexible and/or a co-axial microwave cable and, optionally, wherein the microwave waveguide is housed in the shaft.

## Patentansprüche

1. Mikrowellen-Antennenvorrichtung zur Verwendung beim Abstrahlen von Mikrowellenenergie in Oberflächengewebe eines Gebärmutterhalses, wobei die Mikrowellen-Antennenvorrichtung Folgendes umfasst:
ein elektrisch leitfähiges Erdungselement (31), das eine Öffnung definiert;
ein elektrisch leitfähiges längliches Element (30), das sich durch die Öffnung erstreckt und an einem distalen Ende endet; und
ein oder mehrere dielektrische Elemente (29, 32, 33, 34, 35),
wobei eines oder mehrere der dielektrischen Elemente (29, 33) das längliche Element (30) und das Erdungselement (31) elektrisch voneinander isolieren,
wobei das eine oder die mehreren der dielektrischen Elemente (33, 34) eine Außenfläche der Mikrowellen-Antennenvorrichtung zum Eingriff mit einer Oberfläche des Gebärmutterhalses definieren,
wobei das eine oder die mehreren dielektrischen Elemente ein mittiges distales Merkmal zum Zentrieren der Mikrowellen-Antennenvorrichtung in Bezug auf eine Achse eines Muttermundes des Gebärmutterhalses definieren, und
wobei das eine oder die mehreren dielektrischen Elemente (33, 35) ein Ansaugmerkmal zum Ansaugen eines proximalen Ektozervixbereichs des Gebärmutterhalses und Verhindern eines übermäßigen Einführens des mittigen distalen Merkmals in den Muttermund des Gebärmutterhalses definieren.

2. Mikrowellen-Antennenvorrichtung nach Anspruch 1, wobei das Erdungselement und das längliche Element koaxial sind.

3. Mikrowellen-Antennenvorrichtung nach einem der vorhergehenden Ansprüche, wobei eines oder mehrere der dielektrischen Elemente das distale Ende des länglichen Elements abdecken und/oder wobei eines oder mehrere der dielektrischen Elemente einen distalen Abschnitt des länglichen Elements abdecken.

4. Mikrowellen-Antennenvorrichtung nach einem der vorhergehenden Ansprüche, wobei sich das längliche Element axial um eine vorbestimmte Länge über das Erdungselement hinaus erstreckt und, wahlweise, wobei eines oder mehrere der dielektrischen Elemente einen Teil der vorbestimmten Länge des länglichen Elements abdecken oder eines oder mehrere der dielektrischen Elemente die Gesamtheit der vorbestimmten Länge des länglichen Elements abdecken.

5. Mikrowellen-Antennenvorrichtung nach einem der vorhergehenden Ansprüche, wobei eines oder mehrere der dielektrischen Elemente so konfiguriert sind, dass sie verhindern, dass das längliche Element in Berührung mit der Oberfläche des Gebärmutterhalses kommt, wenn die Mikrowellen-Antennenvorrichtung in Verwendung ist.

6. Mikrowellen-Antennenvorrichtung nach einem der vorhergehenden Ansprüche, wobei eines oder mehrere der dielektrischen Elemente dafür konfiguriert sind, das Erdungselement und/oder das längliche Element um einen gewünschten vorbestimmten Abstand von dem Gewebe der Oberfläche des Gebärmutterhalses zu trennen, zum Beispiel, wobei eines oder mehrere der dielektrischen Elemente eine gewünschte vorbestimmte Dicke aufweisen.

7. Mikrowellen-Antennenvorrichtung nach einem der vorhergehenden Ansprüche, wobei das eine oder die mehreren dielektrischen Elemente die Öffnung ausfüllen, die durch das Erdungselement definiert ist.

8. Mikrowellen-Antennenvorrichtung nach Anspruch 1, wobei das mittige distale Merkmal zum Abstrahlen von Mikrowellenenergie in eine proximale Sektion des Muttermundes konfiguriert ist.

9. Mikrowellen-Antennenvorrichtung nach einem der vorhergehenden Ansprüche, wobei eines oder mehrere der dielektrischen Elemente mindestens einen Teil des Erdungselements abdecken und/oder wobei eines oder mehrere der dielektrischen Elemente mindestens einen Teil einer distalen Oberfläche des Erdungselements abdecken.

10. Mikrowellen-Antennenvorrichtung nach einem der vorhergehenden Ansprüche, wobei die Mikrowellen-Antennenvorrichtung eine Achse definiert und die Mikrowellen-Antennenvorrichtung zylindersymmetrisch um die Achse ist und, wahlweise, wobei das längliche Element stabartig, zylindrisch und/oder kegelförmig ist und, wahlweise, wobei eine radiale Ausdehnung des Erdungselements um einen vorbestimmten radialen Versatz mehr als oder weniger als eine radiale Ausdehnung des einen oder der mehreren dielektrischen Elemente beträgt und, wahlweise, wobei mindestens eines von Folgendem gilt:
das Erdungselement ist ringförmig oder im Allgemeinen ringförmig;
das Erdungselement ist eben oder im Allgemeinen eben;
das Erdungselement ist gekrümmt;
das Erdungselement ist in der Form eines Napfs oder einer Schale geformt, wobei eine Öffnung des Napfs oder der Schale hin zu dem distalen Ende des länglichen Elements gerichtet ist; und
das Erdungselement ist in der Form eines umgekehrten Napfs oder einer umgekehrten Schale geformt, wobei eine Öffnung des umgekehrten Napfs oder der umgekehrten Schale weg von dem distalen Ende des länglichen Elements gerichtet ist.

11. Mikrowellen-Antennenvorrichtung nach einem der vorhergehenden Ansprüche, die ein elektrisch leitfähiges Kappenelement an dem distalen Ende des länglichen Leiters oder angrenzend an dasselbe umfasst, und, wahlweise, wobei ein distales Ende einer Außenfläche der Mikrowellen-Antennenvorrichtung durch eines oder mehrere der dielektrischen Elemente definiert ist und sich das elektrisch leitfähige Kappenelement zwischen dem distalen Ende des länglichen Leiters und dem distalen Ende der Außenfläche der Mikrowellen-Antennenvorrichtung befindet.

12. Mikrowellen-Antennenvorrichtung nach einem der vorhergehenden Ansprüche, wobei die Mikrowellen-Antennenvorrichtung zur Verwendung beim Abstrahlen von Mikrowellenenergie in das Oberflächengewebe des Gebärmutterhalses für mindestens eines von Folgendem konfiguriert ist:
um so eine therapeutische Wirkung in einem oder mehreren Bereichen, wie beispielsweise einem oder mehreren Bereichen des Gebärmutterhalses, die mit dem Humanen Papillomavirus (HPV) infiziert sind und/oder bei denen Zervikale intraepitheliale Neoplasie (CIN) diagnostiziert ist, bereitzustellen;
um so die richtige biologische Reaktion in einem oder mehreren Bereichen, wie beispielsweise einem oder mehreren Bereichen des Gebärmutterhalses, die mit dem Humanen Papillomavirus (HPV) infiziert sind und/oder bei denen Zervikale intraepitheliale Neoplasie (CIN) diagnostiziert ist, zu erzeugen;
örtliche nicht-ablative Hyperthermie des Oberflächengewebes des Gebärmutterhalses;
örtliche Ablation des Oberflächengewebes des Gebärmutterhalses, und
Kauterisation des Oberflächengewebes des Gebärmutterhalses.

13. Vielzahl von Mikrowellen-Antennenvorrichtungen nach einem der vorhergehenden Ansprüche, wobei jede Mikrowellen-Antennenvorrichtung eine unterschiedliche Konfiguration aufweist, die ausgewählt ist, um ein entsprechendes unterschiedliches Abstrahlungsmuster bereitzustellen.

14. Mikrowellenbaugruppe zum Abstrahlen von Mikrowellenenergie in Oberflächengewebe eines Gebärmutterhalses, wobei die Mikrowellenbaugruppe eine Mikrowellen-Antennenvorrichtung nach einem der vorhergehenden Ansprüche und einen Schaft umfasst, der mit der Mikrowellen-Antennenvorrichtung verbunden ist, und, wahlweise, eine Verbindungsanordnung umfasst, welche den Schaft und die Mikrowellen-Antennenvorrichtung verbindet, wobei die Verbindungsanordnung dafür konfiguriert ist, einen Winkel zwischen einer Achse der Mikrowellen-Antennenvorrichtung und einer Achse des Schafts zu verändern, und, wahlweise, wobei die Verbindungsanordnung mindestens eines von einer Drehgelenkanordnung, einem Scharnier, einem flexiblen Gelenk und einem Kugelgelenk umfasst und, wahlweise, wobei die Verbindungsanordnung dafür konfiguriert ist, die Mikrowellen-Antennenvorrichtung abnehmbar an dem Schaft anzubringen, um dadurch das Einpassen einer unterschiedlichen Mikrowellen-Antennenvorrichtung an dem Schaft zu ermöglichen, wobei die unterschiedliche Mikrowellen-Antennenvorrichtung eine alternative Konfiguration, zum Beispiel eine alternative Form und/oder Größe, gegenüber der Mikrowellen-Antennenvorrichtung aufweist, und, wahlweise, wobei der Schaft ein Einwegartikel ist oder der Schaft wiederverwendbar ist und, wahlweise, einen Handgriff oder ein Handstück an oder angrenzend an einem proximalen Ende davon, zum Beispiel an oder angrenzend an einem proximalen Ende des Schafts, umfasst und, wahlweise, wobei der Handgriff oder das Handstück abnehmbar an dem Schaft angebracht ist und, wahlweise, wobei der Handgriff oder das Handstück ein Einwegartikel oder wiederverwendbar ist und, wahlweise, einen elektrischen Schalter umfasst, der konfigurierbar ist zwischen einem Ein-Zustand, in dem der Schalter die Übertragung von Mikrowellenenergie von einem Mikrowellenerzeuger zu der Mikrowellen-Antennenvorrichtung ermöglicht, und einem Aus-Zustand, in dem der Schalter die Übertragung von Mikrowellenenergie von dem Mikrowellenerzeuger zu der Mikrowellen-Antennenvorrichtung verhindert, und, wahlweise, wobei der Handgriff oder das Handstück ein manuelles Steuerungselement, wie beispielsweise einen Knopf oder dergleichen, zum Rekonfigurieren des elektrischen Schalters zwischen dem Ein- und dem Aus-Zustand umfasst.

15. Mikrowellensystem zum Abstrahlen von Mikrowellenenergie in Oberflächengewebe eines Gebärmutterhalses, wobei das System Folgendes umfasst:
eine Mikrowellenbaugruppe nach einem der Ansprüche 1 bis 14;
einen Mikrowellenerzeuger; und
einen Mikrowellen-Wellenleiter,
wobei der Mikrowellen-Wellenleiter den Mikrowellenerzeuger und die Mikrowellenbaugruppe elektrisch verbindet und, wahlweise, wobei der Mikrowellen-Wellenleiter ein Mikrowellenkabel, wie beispielsweise ein flexibles und/oder ein koaxiales Mikrowellenkabel, umfasst und, wahlweise, wobei der Mikrowellen-Wellenleiter in dem Schaft untergebracht ist.

## Revendications

1. Appareil d'antenne hyperfréquence pour utilisation dans le rayonnement d'énergie hyperfréquence dans un tissu de surface d'un col de l'utérus, l'appareil d'antenne hyperfréquence comprenant :
un élément de masse conducteur d'électricité (31) définissant une ouverture ;
un élément allongé conducteur d'électricité (30) s'étendant à travers l'ouverture et se terminant au niveau d'une extrémité distale ; et
un ou plusieurs éléments diélectrique (29, 32, 33, 34, 35),
dans lequel un ou plusieurs des éléments diélectriques (29, 33) isolent électriquement l'élément allongé (30) et l'élément de masse (31) l'un de l'autre,
dans lequel un ou plusieurs des éléments diélectriques (33, 34) définissent une surface externe de l'appareil d'antenne hyperfréquence pour l'engagement dans une surface du col de l'utérus,
dans lequel l'un ou les plusieurs éléments diélectriques définissent une caractéristique distale centrale pour centrer l'appareil d'antenne hyperfréquence par rapport à un axe d'un orifice du col de l'utérus, et
dans lequel l'un ou les plusieurs éléments diélectriques (33, 35) définissent une caractéristique de ventouse pour appliquer une ventouse sur une région proximale de l'exocol du col de l'utérus et empêcher une insertion excessive de la caractéristique distale centrale dans l'orifice du col de l'utérus.

2. Appareil d'antenne hyperfréquence selon la revendication 1, dans lequel l'élément de masse et l'élément allongé sont coaxiaux.

3. Appareil d'antenne hyperfréquence selon l'une quelconque des revendications précédentes, dans lequel un ou plusieurs des éléments diélectriques recouvrent l'extrémité distale de l'élément allongé, et/ou dans lequel un ou plusieurs des éléments diélectriques recouvrent une partie distale de l'élément allongé.

4. Appareil d'antenne hyperfréquence selon l'une quelconque des revendications précédentes, dans lequel l'élément allongé s'étend axialement au-delà de l'élément de masse d'une longueur prédéterminée et, dans lequel un ou plusieurs des éléments diélectriques recouvrent optionnellement une partie de la longueur prédéterminée de l'élément allongé ou un ou plusieurs des éléments diélectriques recouvrent la totalité de la longueur prédéterminée de l'élément allongé.

5. Appareil d'antenne hyperfréquence selon l'une quelconque des revendications précédentes, dans lequel un ou plusieurs des éléments diélectriques sont configurés pour empêcher une entrée en contact de l'élément allongé avec la surface du col de l'utérus lorsque l'appareil d'antenne hyperfréquence est en cours d'utilisation.

6. Appareil d'antenne hyperfréquence selon l'une quelconque des revendications précédentes, dans lequel un ou plusieurs des éléments diélectriques sont configurés pour séparer l'élément de masse et/ou l'élément allongé du tissu de la surface du col de l'utérus d'une distance prédétermine souhaitée, dans lequel un ou plusieurs des éléments diélectriques présentent par exemple une épaisseur prédéterminée souhaitée.

7. Appareil d'antenne hyperfréquence selon l'une quelconque des revendications précédentes, dans lequel l'un ou les plusieurs éléments diélectriques remplissent l'ouverture définie par l'élément de masse.

8. Appareil d'antenne hyperfréquence selon la revendication 1, dans lequel la caractéristique distale centrale est configurée pour rayonner de l'énergie hyperfréquence dans une section proximale de l'orifice du col de l'utérus.

9. Appareil d'antenne hyperfréquence selon l'une quelconque des revendications précédentes, dans lequel un ou plusieurs des éléments diélectriques recouvrent au moins une partie de l'élément de masse, et/ou dans lequel un ou plusieurs des éléments diélectriques recouvrent au moins une partie d'une surface distale de l'élément de masse.

10. Appareil d'antenne hyperfréquence selon l'une quelconque des revendications précédentes, dans lequel l'appareil d'antenne hyperfréquence définit un axe et l'appareil d'antenne hyperfréquence est cylindriquement symétrique autour de l'axe et, dans lequel l'élément allongé a optionnellement une forme de tige, cylindrique et/ou conique, et dans lequel une étendue radiale de l'élément de masse est optionnellement supérieure ou inférieure à une étendue radiale de l'un ou des plusieurs éléments diélectriques, d'un décalage radial prédéterminé, et optionnellement, dans lequel au moins une des conditions suivantes s'applique :
l'élément de masse est annulaire ou généralement annulaire ;
l'élément de masse est plan ou généralement plan ;
l'élément de masse est courbé ;
l'élément de masse présente la forme d'une coupelle ou d'un bol, avec une ouverture de la coupelle ou du bol dirigée vers l'extrémité distale de l'élément allongé, ; et
l'élément de masse présente la forme d'une coupelle inversée ou d'un bol inversé, avec une ouverture de la coupelle inversée ou du bol inversé dirigée à l'écart d l'extrémité distale de l'élément allongé.

11. Appareil d'antenne hyperfréquence selon l'une quelconque des revendications précédentes, comprenant un élément de capuchon conducteur d'électricité au niveau de ou adjacent à l'extrémité distale du conducteur allongé, et dans lequel une extrémité distale d'une surface externe de l'appareil d'antenne hyperfréquence est optionnellement définie par un ou plusieurs des éléments diélectriques, et l'élément de capuchon conducteur d'électricité est situé entre l'extrémité distale du conducteur allongé et l'extrémité distale de la surface externe de l'appareil d'antenne hyperfréquence.

12. Appareil d'antenne hyperfréquence selon l'une quelconque des revendications précédentes, dans lequel l'appareil d'antenne hyperfréquence est configuré pour utilisation dans le rayonnement de l'énergie hyperfréquence dans le tissu de surface du col d l'utérus, pour au moins un des éléments suivants :
pour fournir un effet thérapeutique dans une ou plusieurs régions, par exemple une ou plusieurs régions du col de l'utérus infectées par le papillomavirus humain (HPV) et/ou diagnostiquées avec une néoplasie intraépithéliale cervicale (CIN) ;
pour créer la réponse biologique correcte dans une ou plusieurs régions, par exemple une ou plusieurs régions du col de l'utérus infectées par le papillomavirus humain (HPV) et/ou diagnostiquées avec une néoplasie intraépithéliale cervicale (CIN) ;
une hyperthermie non ablative localisée du tissu de surface du col de l'utérus ;
une ablation localisée du tissu de surface du col de l'utérus ; et
une cautérisation du tissu de surface du col de l'utérus.

13. Pluralité d'appareils d'antenne hyperfréquence selon l'une quelconque des revendications précédentes, dans laquelle chaque appareil d'antenne hyperfréquence a une configuration différente sélectionnée pour fournir un motif de rayonnement différent correspondant.

14. Ensemble hyperfréquence pour rayonner de l'énergie hyperfréquence dans un tissu de surface d'un col de l'utérus, l'ensemble hyperfréquence comprenant l'appareil d'antenne hyperfréquence selon l'une quelconque des revendications précédentes, et un arbre relié à l'appareil d'antenne hyperfréquence et, comprenant optionnellement un agencement de connexion connectant l'arbre et l'appareil d'antenne hyperfréquence, dans lequel l'agencement de connexion est configuré pour changer un angle entre un axe de l'appareil d'antenne hyperfréquence et un axe de l'arbre, et dans lequel l'agencement de connexion comprend optionnellement au moins un parmi un agencement de pivot, un charnière, un joint flexible et un joint à rotule, et dans lequel l'agencement de connexion est optionnellement configuré pour fixer de manière amovible l'appareil d'antenne hyperfréquence sur l'arbre pour permettre ainsi l'ajustement d'un appareil d'antenne hyperfréquence différent sur l'arbre, dans lequel l'appareil d'antenne hyperfréquence différent présente une configuration alternative, par exemple une forme et/ou une taille alternative à l'appareil d'antenne hyperfréquence, et dans lequel l'arbre est optionnellement jetable ou l'arbre est réutilisable, et comprenant optionnellement un poignée ou une pièce à main au niveau de ou adjacente à une extrémité proximale de celle-ci, par exemple au niveau de ou adjacente à une extrémité proximale de l'arbre, et, dans lequel la poignée ou la pièce à main est optionnellement fixée de manière amovible sur l'arbre et, dans lequel la poignée ou la pièce à main est optionnellement jetable ou réutilisable, et comprenant optionnellement un interrupteur électrique qui peut être configuré entre un état activé, dans lequel l'interrupteur permet la transmission d'énergie hyperfréquence d'un générateur hyperfréquence à l'appareil d'antenne hyperfréquence, et un état désactivé, dans lequel l'interrupteur empêche la transmission d'énergie hyperfréquence du générateur hyperfréquence à l'appareil d'antenne hyperfréquence, et dans lequel la poignée ou la pièce à main comprend optionnellement un élément de commande manuelle, par exemple un bouton ou similaire, pour reconfigurer l'interrupteur électrique entre les états activé et désactivé.

15. Système hyperfréquence pour rayonner de l'énergie hyperfréquence dans un tissu de surface d'un col de l'utérus, le système comprenant :
un ensemble hyperfréquence selon l'une quelconque des revendications 1 à 14 ;
un générateur hyperfréquence ; et
un guide d'ondes hyperfréquence,
dans lequel le guide d'ondes hyperfréquence connecte électriquement le générateur hyperfréquence et l'ensemble hyperfréquence, et dans lequel le guide d'ondes hyperfréquence comprend optionnellement un câble hyperfréquence, par exemple un câble hyperfréquence flexible et/ou coaxial, et dans lequel le guide d'ondes hyperfréquence est optionnellement logé dans l'arbre.
